# FASCICULE DE BREVET EUROPEEN

(11) **EP 1 291 003 B1**
(45) Date de publication et mention de la délivrance du brevet: **21.02.2007**
(21) Numéro de dépôt: 02292002.9
(22) Date de dépôt: 08.08.2002
(51) Int. Cl.: A61K 8/81, A61K 8/895, A61Q 5/00

(54) **Compositions cosmétiques contenant un copolymère d'acide méthacrylique, une silicone et un polymère cationique et leurs utilisations**
Ein Copolymer aus Methacrylsäure, ein Silikon und ein kationisches Polymer enthaltende kosmetische Zusammensetzungen und deren Verwendungen
Cosmetic compositions containing a copolymer of methacrylic acid, a silicone and a cationic polymer and uses thereof

(30) Priorité: 11.09.2001 FR 0111740
(43) Date de publication de la demande: 12.03.2003
(73) Titulaire: L'ORÉAL, 75008 Paris (FR)
(72) Inventeur: Maubru, Mireille, 78400 Chatou (FR); Liebard, Bruno, 92320 Chatillon (FR)
(74) Mandataire: Le Blainvaux Bellegarde, Françoise

(56) Documents cités:
- EP-A- 1 138 315
- WO-A-98/03155
- DE-A- 19 750 520
- FR-A- 2 739 289

## Description

La présente invention concerne de nouvelles compositions cosmétiques comprenant dans un milieu cosmétiquement acceptable au moins un copolymère acide méthacrylique / acrylate d'alkyle en C₁-C₄ réticulé, au moins un polymère cationique ou amphotère et au moins une silicone fonctionnalisée.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou amphotères ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).
En outre, l'usage des polymères cationiques ou amphotères dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

Les silicones fonctionnalisées sont généralement utilisées dans les compositions de shampooings en tant qu'agents conditionneurs pour améliorer la douceur, le toucher et le démêlage des cheveux. Cependant, on a constaté que ces silicones conduisaient à la formation d'une couche inesthétique à la surface du shampooing nuisible aux performances du shampooing. Pour éviter l'apparition de ce phénomène, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

La demande de brevet FR 2 739 289 décrit des compositions cosmétiques comprenant au moins un polymère greffé et au moins une association d'un polymère anionique et d'un polymère cationique.

En résumé, il s'avère que les compositions cosmétiques actuelles contenant des polymères cationiques ou amphotères, ne donnent pas encore complètement satisfaction.

On connaît dans l'état de la technique des compositions cosmétiques en particulier détergentes contenant un copolymère d'acide méthacrylique et d'acrylate d'alkyle comme agent de stabilisation ou de suspension d'ingrédients insolubles dans l'eau comme des silicones ou des corps gras. De telles compositions ont été décrites notamment dans la demande de brevet WO01/76552. Les qualités de mousse et les propriétés cosmétiques obtenues avec ces compositions ne sont pas encore suffisamment satisfaisantes.

La demanderesse a maintenant découvert que l'association d'un copolymère réticulé acide méthacrylique / acrylate d'alkyle en C₁-C₄, d'un polymère cationique ou amphotère et d'une silicone particulière permet de remédier à ces inconvénients.
En effet, il a été constaté que l'utilisation dudit copolymère acrylique dans les compositions de la présente invention permettait d'obtenir sur les matières kératiniques, notamment les cheveux de très bonnes propriétés cosmétiques particulièrement au niveau de la légèreté, de la douceur, du lissage au toucher, au niveau de la souplesse et de la malléabilité sur cheveux séchés. Il a aussi été constaté que les compositions de l'invention permettaient d'obtenir des cheveux séchés présentant au regard un aspect général plus lisse.

Par ailleurs, les compositions selon l'invention sont stables et présentent un aspect visuel esthétique. Les propriétés d'usage (aspect, consistance, abondance de la mousse, élimination de la mousse) sont très satisfaisantes.

Les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un copolymère d'acide méthacrylique et d'acrylate d'alkyle en C₁-C₄ réticulé, au moins un polymère cationique ou amphotère et au moins une silicone choisie parmi :
(i) les polyorganosiloxanes, comportant dans leur structure générale, un ou plusieurs groupements organofonctionnels directement fixés sur la chaîne siloxanique ou fixés par l'intermédiaire d'un radical hydrocarboné, à l'exclusion des silicones solubles choisies parmi les polydiméthylsiloxanes comportant des groupements polyoxyéthylénés terminaux et/ou latéraux;
(ii) les copolymères blocs linéaires polysiloxane(A)-polyoxyalkylène(B) de type (A-B)n avec n >3 ;
(iii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(iv) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(v) ou leurs mélanges.

L'invention a également pour objet l'utilisation d'une composition telle que définie ci-dessus pour apporter de la brillance, de la légèreté, de la douceur, du lissage au toucher, de la souplesse aux cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.

Un autre objet de l'invention concerne l'utilisation d'un copolymère réticulé acide méthacrylique / acrylate d'alkyle en C₁-C₄ dans, ou pour la fabrication d'une composition cosmétique comprenant un polymère cationique ou amphotère et une silicone particulière telle que définie ci-dessous.

Par insolubles dans l'eau, on comprend selon l'invention les composés insolubles dans l'eau à une concentration supérieure ou égale à 0,1% en poids dans l'eau à 25°C, c'est à dire qu'ils ne forment pas une solution macroscopiquement isotrope et transparente.

Les différents objets de l'invention vont maintenant être détaillés. L'ensemble des significations et définitions des composés utilisés dans la présente invention données ci-dessous sont valables pour l'ensemble des objets de l'invention.

L'une des caractéristiques essentielles de l'invention est la présence d'un copolymère d'acide méthacrylique et d'acrylate d'alkyle en C₁-C₄, réticulé.

L'acide méthacrylique est présent de préférence dans des quantités allant de 20 à 80% en poids et plus particulièrement de 25 à 70 % en poids et encore plus particulièrement de 35 à 60% en poids par rapport au poids total du copolymère.

L'acrylate d'alkyle est présent de préférence dans des quantités allant de 15 à 80% en poids et plus particulièrement de 25 à 75 % en poids et encore plus particulièrement de 40 à 65% en poids par rapport au poids total du copolymère. Il est choisi notamment parmi l'acrylate de méthyle, l'acrylate d'éthyle ou l'acrylate de butyle et plus particulièrement l'acrylate d'éthyle.

Ce copolymère est partiellement ou totalement réticulé par au moins un agent réticulant classique. Les agents réticulants sont notamment des composés polyinsaturés, en particulier éthylèniquement polyinsaturés. Ces composés sont notamment les polyalcényléthers de sucrose ou de polyols, les diallylphtalates, le divinylbenzene, le (méth)acrylate d'allyle, di(méth)acrylate d'éthylèneglycol, le méthylène bis-acrylamide, le tri(méth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés.
Comme agent réticulant, on peut également utiliser des composés monomères insaturés et comportant un groupe réactif susceptible de réagir avec une insaturation pour former un copolymère réticulé.
La teneur en agent réticulant varie en général de 0,01 à 5% en poids et de préférence de 0,03 à 3% en poids et encore plus particulièrement de 0,05 à 1% en poids par rapport au poids total du copolymère.

Selon une forme particulièrement préférée, le copolymère de l'invention peut se présenter notamment sous forme de dispersion dans l'eau. La taille moyenne en nombre des particules de copolymère dans la dispersion est généralement comprise entre 10 et 500 nm et de préférence entre 20 et 200 nm et plus préférentiellement de 50 à 150 nm.

Ces copolymères sont notamment décrit dans la demande WO01/76552.

On utilisera plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.

La concentration en copolymère est généralement comprise entre 0,01 et 20% en poids, entre 0,01 et 10% en poids par rapport au poids total de la composition et de préférence entre 0,1 et 5% en poids.

Les silicones utilisables conformément à l'invention sont en particulier insolubles dans la composition et peuvent se présenter sous forme d'huiles, de cires, de résines ou de gommes.

Les silicones sont définies plus en détail dans l'ouvrage de Walter NOLL "Chemistry and Technology of Silicones" (1968) Academie Press.

On utilise de préférence des silicones non volatiles. Par silicone non volatile, on entend selon la présente invention, toute silicone présentant une pression de vapeur mesurée à 25°C à la pression atmosphérique (10⁵ Pa) de préférence inférieure à 0,01 mm Hg (2,6 Pa).

Les polyorganosiloxanes comportant un ou plusieurs groupements organofonctionnels de l'invention sont, par exemple, les polysiloxanes notamment polydiméthylsiloxanes comportant
a) des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 939 par la société DOW CORNING. Les groupements aminés substitués sont en particulier des groupements aminoalkyle en C₁-C₄ et aminoalkyle en C₁-C₄iminoalkyle en C₁-C₄. On utilise plus particulièrement les silicones dénommées amodiméthicone et triméthylsilylamodiméthicone selon la dénomination CTFA ;
b) des groupements (per)fluorés, comme les groupements trifluoroalkyles tels que, par exemple, ceux vendus par SHIN ETSU sous le nom FL 100 ;
c) des groupements thiols ;
d) des groupements carboxylates, tels que les produits décrits dans le brevet européen EP 185 507 de CHISSO CORPORATION ;
e) des groupements hydroxylés, tels que les polyorganopolysiloxanes à fonction hydroxyalkyle décrits dans la demande de brevet français FR 85-16334 et en particulier les polyorganopolysiloxanes à fonction γ- hydroxypropyle ;
f) des groupements alcoxylés comportant au moins 12 atomes de carbone, tels que le produit SILICONE COPOLYMER F755 de SWS SILICONES et les produits ABILWAX® 2428, ABILWAX® 2434, ABILWAX® 2440 de la société GOLDSCHMIDT.
g) des groupements acyloxyalkyles comportant au moins 12 atomes de carbone, tels que les polyorganosiloxanes décrits dans la demande de brevet français FR 88-17433 et en particulier les polyorganosiloxanes à fonction stéaroyloxypropyle.
h) des groupements amphotères;
i) des groupements bisulfites.
j) des groupements hydroxyacylamino, comme les polyorganosiloxanes décrits dans la demande EP 342 834. On peut citer par exemple le produit Q2-8413 de la société DOW CORNING.
k) des groupements acide carboxylique ou leur sels tels que les produits commercialisés par BASF sous la dénomination DENSODRIN OF ou par WACKER sous la dénomination huile MS642.
l) des groupements aryles

Ils sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF1265.

Les polyorganosiloxanes organomodifiés de l'invention ne comportent pas de préférence de groupements polyalkylénés et notamment polyoxyéthylénés.

Les copolymères blocs ayant un bloc linéaire polysiloxane-polyoxyalkylène de type (A-B)n utilisés dans le cadre de la présente invention ont de préférence la formule générale suivante :

([ Y (R₂SiO)ₐ R'₂SiYO] [(CnH₂ₙO)_{b}])_{c} (I)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,
- le poids molaire moyen de chaque bloc siloxane est compris entre environ 400 et environ 10.000, celui de chaque bloc polyoxyalkylène étant compris entre environ 300 et environ 10.000,
- les blocs siloxane représentent de 10 % environ à 95 % environ en poids du copolymère bloc,
- le poids molaire moyen en poids du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyles comme par exemple les radicaux méthyle, éthyle, propyle, butyle, pentyle, hexyle, octyle, décyle, dodécyle, les radicaux aryles comme par exemple phényle, naphtyle, les radicaux aralkyles comme par exemple benzyle, phényléthyle, les radicaux tolyle, xylyle et cyclohexyle.

Y est de préférence -R"-, -R"-CO-, -R"-NHCO-, -R"-NH-CO-NH-R"'-NHCO, -R"-OCONH-R'''-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1, dont l'enseignement est inclus à titre de référence dans la présente description.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578 dont les enseignements sont totalement inclus dans la présente description à titre de références non limitatives. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Des polymères à squelette polysiloxanique greffé par des monomères organiques non siliconés convenant à la mise en oeuvre de la présente invention, ainsi que leur mode particulier de préparation, sont notamment décrits dans les demandes de brevets EP-A- 0 582 152, WO 93/23009 et WO 95/03776 dont les enseignements sont totalement inclus dans la présente description à titre de références non limitatives.

Comme exemple de polymères, on peut citer le produit dénommé POLYSILICONE 8 dans la 7^{ème} édition de l'ouvrage International Cosmetic Ingredients Dictionary and Handbook.

Selon l'invention, toutes les silicones peuvent être utilisées telle quelle ou sous forme d'émulsions, de nanoémulsions ou de microémulsions.

Les silicones particulièrement préférées conformément à l'invention sont :
- les polysiloxanes à groupements aminés tels que les amodiméthicones ou les triméthylsilylamodiméthicones (CTFA 4^{ème} édition 1997) ;

La ou les silicones sont utilisées de préférence en une quantité comprise entre 0,001 et 20% en poids par rapport au poids total de la composition. Plus préférentiellement, cette quantité est comprise entre 0,05 et 15% en poids par rapport au poids total de la composition et encore plus préférentiellement entre 0,1 et 10% en poids.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 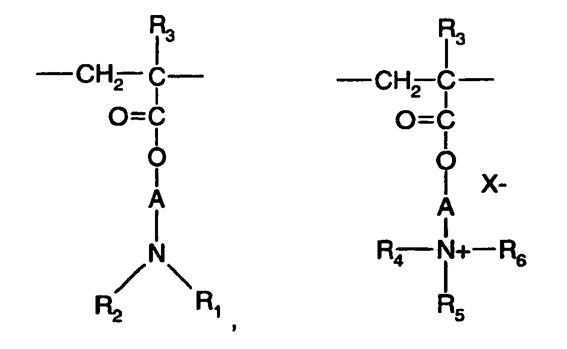 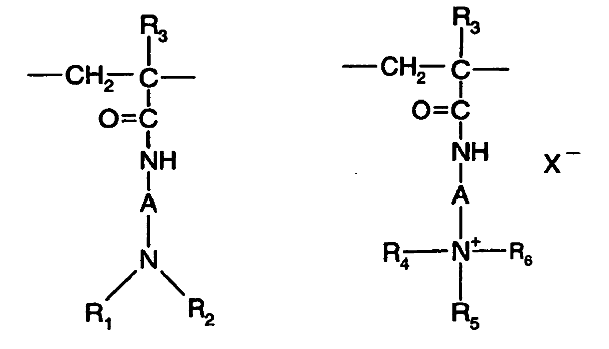 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R_{6,} identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate
      ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société NALCO. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement aminé du polyaminoamide; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (II) ou (III) : 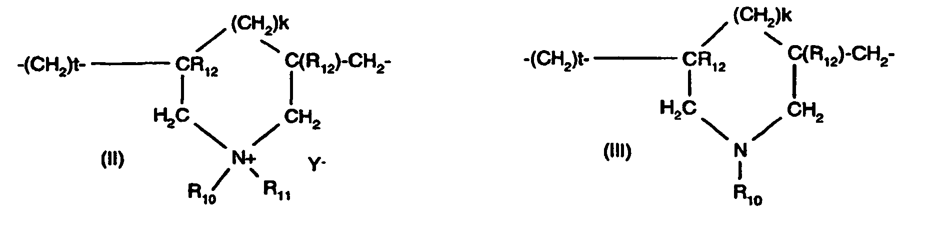 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide commercialisés sous la dénomination "MERQUAT 550".
(8) les polymères de diammonium quaternaire contenant des motifs récurrents répondant à la formule : 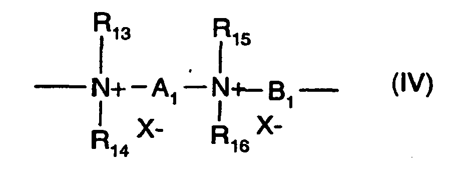 formule (IV) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;
   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 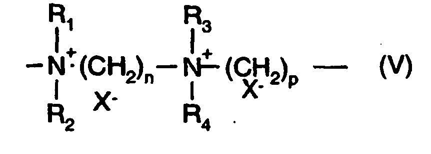 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (VI): 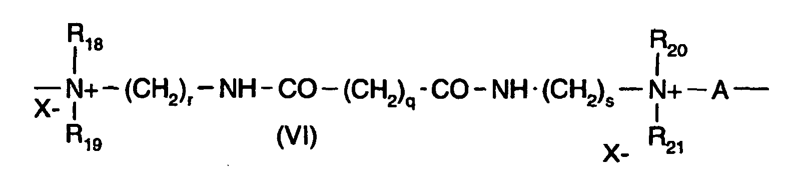 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société NALCO, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléfique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société NALCO.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅ CO―R₄―CO―Z⁆ (VII)

   dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical de formule (VIII) 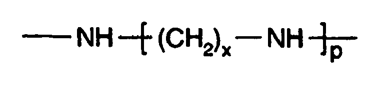 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (VIII) ci-dessus, dans lequel x=2 et p=1 et qui dérive de l'éthylènediamine, ou le radical dérivant de la pipérazine : 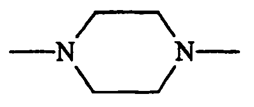
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule (IX): 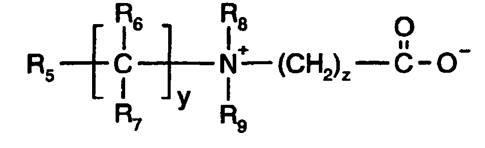 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle/méthacrylate de diméthyl-carboxyméthylammonio-éthyl tel que le produit vendu sous la dénomination DIAFORMER Z301 par la société SANDOZ.
(5) les polymères dérivés du chitosane comportant des motifs monomères répondant aux formules (X), (XI), (XII) suivantes : 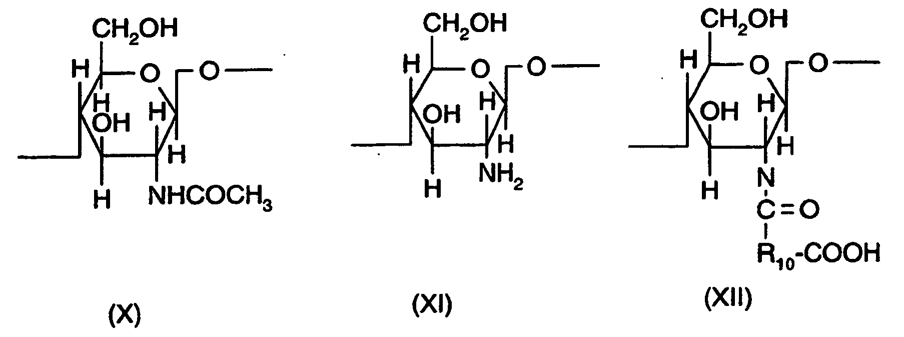 le motif (X) étant présent dans des proportions comprises entre 0 et 30%, le motif (XI) dans des proportions comprises entre 5 et 50% et le motif (XII) dans des proportions comprises entre 30 et 90%, étant entendu que dans ce motif (XII), R₁₀ représente un radical de formule : 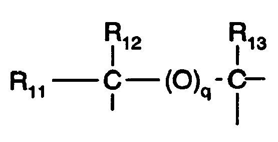 dans laquelle
   si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XIII) tels que ceux décrits par exemple dans le brevet français 1 400 366 : 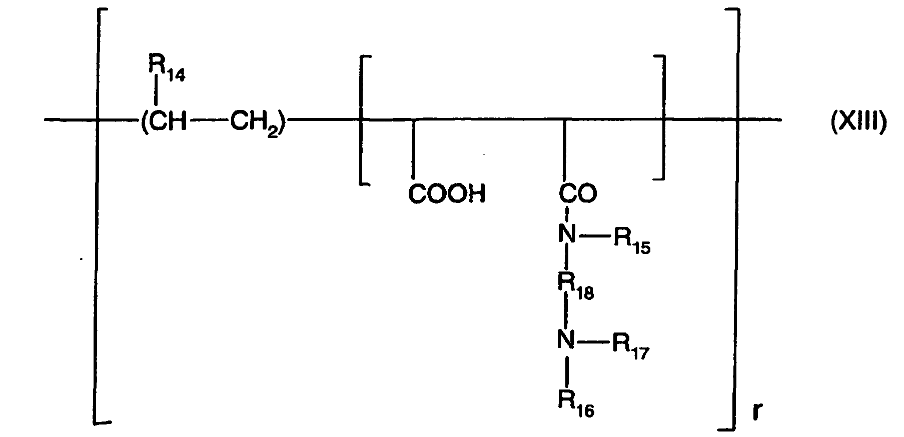 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XIV)

      où D désigne un radical 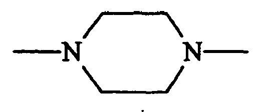 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (VX)
   où D désigne un radical 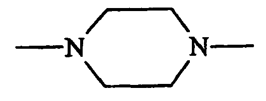 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique
   ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1), notamment les copolymères de sel (par exemple halogénure) de diméthyldiallylammonium et d'acide acrylique.

Selon l'invention, le ou les polymères cationiques ou amphotères peuvent représenter de 0,001 % à 20 % en poids, de préférence de 0,01 % à 10% en poids et plus particulièrement de 0,1 à 5% en poids par rapport au poids total de la composition finale.

Selon un mode de réalisation particulier, les compositions selon l'invention comprennent en outre au moins une silicone autres que celles de l'invention ou un autre agent bénéfique pour les matières kératiniques en particulier les cheveux tels que notamment les esters d'acides carboxyliques en C1-C30 et d'alcools mono ou polyhydroxylés en C1-C30, les huiles végétales, animales, minérales ou de synthèse, les cires, les céramides, les pseudocéramides.

Les silicones additionnelles utilisables conformément à l'invention sont en particulier des polyorganosiloxanes insolubles dans la composition et peuvent se présenter sous forme d'huiles, de cires, de résines ou de gommes.

Ces organopolysiloxanes additionnels sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones additionnelles sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique :
   avec D : 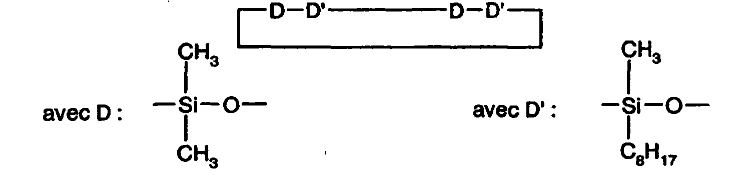 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

Ces silicones additionnelles sont plus particulièrement choisies parmi les polyalkylsiloxanes parmi lesquels on peut citer principalement les polydiméthylsiloxanes à groupements terminaux triméthylsilyle ayant de préférence une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les gommes de silicone utilisables en tant qu'additif sont notamment des polydiorganosiloxanes ayant des masses molaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,

Des produits plus particulièrement utilisables sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids molaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables en tant qu'additif sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Selon l'invention, toutes les silicones peuvent être utilisées tel quel ou sous forme de solutions, d'émulsions, de nanoémulsions ou de microémulsions.

Les silicones particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 cSt, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- les résines d'organopolysiloxans telles que
- la résine d'organopolysiloxane commercialisée sous la dénomination DOW CORNING 593 ;
- les mélanges de deux PDMS constitués d'une gomme et d'une huile de viscosités différentes, les mélanges d'organosiloxanes et de silicones cycliques.

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah
ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols
ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
Parmi les alkyl (C₈-C₂₀) amidoalkyl (C₁-C₆) bétaïnes on peut citer la cocoamidopropylbétaïne vendue notamment par GOLDSCHMIDT sous le nom de TEGOBETAINE F50.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.
(iv) Les tensioactifs cationiques peuvent être choisis parmi :
   A) les sels d'ammonium quaternaires de la formule générale (XVI) suivante : 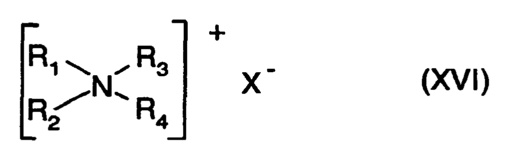
dans laquelle X est un anion choisi dans le groupe des halogénures (chlorure, bromure
ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
, et
a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
   R4 désigne un radical alkyle, linéaire ou ramifié, comportant de 16 à 30 atomes de carbone.
   De préférence le tensioactif cationique est un sel (par exemple chlorure) de béhényl triméthyl ammonium.
b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
   R3 et R4, identiques ou différents, désignent un radical alkyle, linéaire ou ramifié, comportant de 12 à 30 atomes de carbone, ledit radical comprenant au moins une fonction ester ou amide.
   R3 et R4 sont notamment choisis parmi les radicaux alkyl(C₁₂-C₂₂)amido alkyle(C₂-C₆), alkyl(C₁₂-C₂₂)acétate ;
   De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (XVII) suivante : 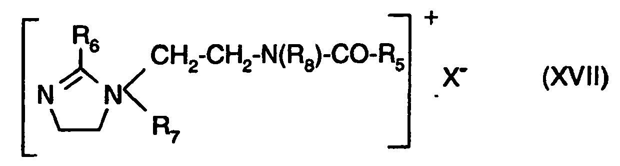 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XVIII) : 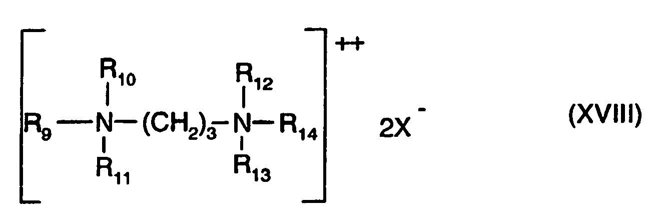 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène
   ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XIX ) suivante : 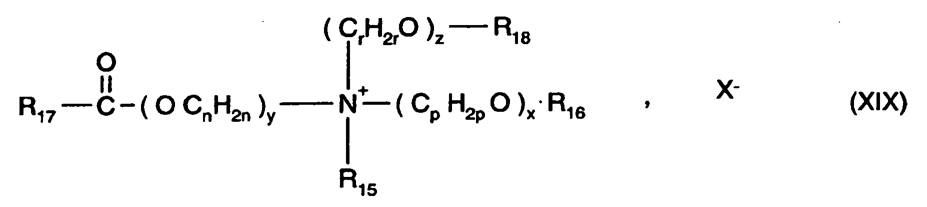 dans laquelle :
   - R₁₅ est choisi parmi les radicaux alkyles en C₁-C₆ et les radicaux hydroxyalkyles ou dihydroxyalkyles en C₁-C₆;
   - R₁₆ est choisi parmi :
      - le radical 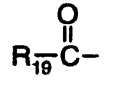
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 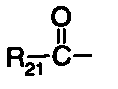
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XIX) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 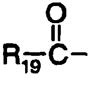
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 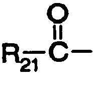
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et au moins un agent tensioactif amphotère.

La composition de l'invention peut également contenir au moins un additif choisi parmi les épaississants, les parfums, les agents nacrants, les conservateurs, les filtres solaires siliconés ou non, les polymères anioniques ou non ioniques non siliconés, les protéines non cationiques, les hydrolysats de protéines non cationiques, l'acide méthyl-18 eicosanoique, les hydroxyacides, les vitamines, les provitamines telles que le panthénol, les agents antipelliculaires ou anti-séborrhéiques, les électrolytes, les esters d'acides gras, les huiles fluorées ou perfluorées, les amines grasses, les acides gras et leurs dérivés, les alcools gras et leurs dérivés ainsi que les mélanges de ces différents composés, et tout autre additif classiquement utilisé dans le domaine cosmétique qui n'affecte pas les propriétés des compositions selon l'invention.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Le milieu physiologiquement et notamment cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol; les alkylèneglycols comme le propylèneglycol, les éthers de glycols.
De préférence, la composition comprend de 50 à 95 % en poids d'eau par rapport au poids total de la composition et plus particulièrement de 60 à 90% en poids.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

Le ou les tensioactifs constituant la base lavante peuvent être indifféremment choisis, seuls ou en mélanges, au sein des tensioactifs anioniques, amphotères, non ioniques et cationiques tels que définis ci-dessus. La base lavante contient au moins un tensioactif détergent.
Dans les compositions conformes à l'invention, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.
Un mélange particulièrement préféré est un mélange comprenant au moins un agent tensioactif anionique et au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes ou les alkylamidobétaïnes et en particulier la cocobétaïne commercialisée sous la dénomination "DEHYTON AB 30" en solution aqueuse à 32 % de MA par la société COGNIS ou la cocoamidopropylbétaïne vendue notamment par GOLDSCHMIDT sous le nom de TEGOBETAINE F50.

La quantité et la qualité de la base lavante sont celles suffisantes pour conférer à la composition finale un pouvoir moussant et/ou détergent satisfaisant.

Ces compositions détergentes sont de préférence moussante et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ainsi, selon l'invention, les tensioactifs détergents peuvent représenter de 3 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, sa concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des matières kératiniques en particulier les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

### EXEMPLES

On a réalisé les shampooings (ex 1 et 2) conformes à l'invention de composition suivante :

| Composition | Exemple 1 | Exemple 2 | Exemple 3 |
|---|---|---|---|
| Lauryléthersulfate de sodium à 2.2 moles d'oxyde d'éthylène | 15 g M.A. | 16 g M.A. | 16 g M.A. |
| Cocoyl amidopropyl bétaïne | 2.4 g M.A. | - | - |
| Cocoyl bétaïne | - | 2.5 g M.A. | 2.4 g M.A. |
| Copolymère réticulé acide méthacrylique / acrylate d'éthyle en émulsion aqueuse à 30% M.A., commercialisé sous la dénomination Carbopol Aqua SF1 par la société NOVEON | 1 g M.A. | 0.9 g M.A. | 0.9 g M.A. |
| Polydiméthylsiloxane de viscosité 60 000 cSt, vendu sous la dénomination DC200 fluid 60 000 cSt par la société DOW CORNING | 1 g | 1.2 g | 1 g |
| Polydiméthylsiloxane à groupements aminoéthyl iminobutyl en émulsion cationique à 60% dans l'eau, commercialisé sous la dénomination DC2-8299 par la société DOW CORNING | | | 2,4 g |
| Polydiméthylsiloxane à groupements aminoéthyl iminopropyl en émulsion cationique à 35% dans l'eau, commercialisé sous la dénomination DC939 par la société DOW CORNING | 4 g | 3 g | - |
| Hydroxyéthylcellulose réticulée à l'épichlorhydrine, quaternisée par la triméthylamine, vendue sous la dénomination JR400 par la société AMERCHOL | - | 0.7 g | |
| Homopolymère de chlorure de diméthyl diallyl ammonium, en solution aqueuse à 40% MA, commercialisé sous la dénomination MERQUAT 100 par la société | 1.2 g | - | 1 g |
| NALCO | | | |
| Mélange 1-(hexadécyloxy) 2-octadécanol / alcool cétylique | - | 2.5 g | |
| Alcool béhénylique | 1.5 g | - | 1.5 g |
| Distéaryl éther | 1.5 g | - | 1.5 g |
| Conservateurs | q.s. | q.s. | q.s. |
| Acide citrique ou soude q.s. | pH 5.5 | pH 7 | pH 5.5 |
| Eau déminéralisée q.s.p. | 100 g | 100 g | 100 g |

Les compositions sont stables. Les cheveux mouillés ne sont pas chargés et la mise en forme est aisée.

### EXEMPLES 3 et 4

On a réalisé 2 shampooings conformes à l'invention de composition suivante :

| Composition | Exemple 3 | Exemple 4 |
|---|---|---|
| Lauryléthersulfate de sodium à 2.2 moles d'oxyde d'éthylène | 16 g M.A. | 15 g M.A. |
| Cocoyl amidopropyl bétaïne | 2.4 g M.A. | - |
| Cocoyl bétaïne | - | 2.4 g M.A. |
| Copolymère réticulé acide méthacrylique / acrylate d'éthyle en émulsion aqueuse à 30% M.A., commercialisé sous la dénomination Carbopol Aqua SF1 par la société NOVEON | 1,2 g M.A. | 1 g M.A. |
| Polydiméthylsiloxane à groupements aminoéthyl iminopropyl en émulsion cationique à 35% dans l'eau, commercialisé sous la dénomination DC939 par la société DOW CORNING | 3 g | 4 g |
| Hydroxyéthylcellulose réticulée à l'épichlorhydrine, quaternisée par la triméthylamine, vendue sous la dénomination JR400 par la société AMERCHOL | 0,7 g | - |
| Homopolymère de chlorure de diméthyl diallyl ammonium, en solution aqueuse à 40% MA, commercialisé sous la dénomination MERQUAT 100 par la société NALCO | - | 1.2 g |
| Mélange 1-(hexadécyloxy) 2-octadécanol / alcool cétylique | 2,5 g | - |
| Alcool béhénylique | - | 1.5 g |
| Distéaryl éther | - | 1.5 g |
| Conservateurs | q.s. | q.s. |
| Acide citrique ou soude q.s. | pH 7 | pH 5,5 |
| Eau déminéralisée q.s.p. | 100 g | 100 g |

La composition est stable. Les cheveux mouillés ne sont pas chargés et la mise en forme est aisée.

## Revendications

1. Composition cosmétique, **caractérisée par le fait qu'**elle comprend, dans un milieu cosmétiquement acceptable, au moins un copolymère réticulé d'acide méthacrylique et d'acrylate d'alkyle en C₁-C₄, au moins un polymère cationique ou amphotère et au moins une silicone choisie parmi :
(i) les polyorganosiloxanes, comportant dans leur structure générale, un ou plusieurs groupements organofonctionnels directement fixés sur la chaîne siloxanique ou fixés par l'intermédiaire d'un radical hydrocarboné à l'exclusion des silicones solubles choisies parmi les polydiméthylsiloxanes comportant des groupements polyoxyéthylénés terminaux et/ou latéraux;
(ii) les copolymères blocs linéaires polysiloxane(A)-polyoxyalkylène(B) de type (A-B)n avec n >3 ;
(iii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(iv) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(v) ou leurs mélanges.

2. Composition selon la revendication 1, **caractérisé en ce que** dans ledit copolymère, l'acide méthacrylique est présent dans des quantités allant de 20 à 80% en poids et plus particulièrement de 25 à 70 % en poids et encore plus particulièrement de 35 à 60% en poids par rapport au poids total du copolymère.

3. Composition selon la revendication 1 ou 2, **caractérisé en ce que** dans ledit copolymère, l'acrylate d'alkyle est présent dans des quantités allant de 15 à 80% en poids et plus particulièrement de 25 à 75 % en poids et encore plus particulièrement de 40 à 65% en poids par rapport au poids total du copolymère.

4. Composition selon l'une quelconque des revendications 1 à 3, **caractérisé en ce que** dans ledit copolymère, l'acrylate d'alkyle est choisi parmi l'acrylate de méthyle, l'acrylate d'éthyle ou l'acrylate de butyle.

5. Composition selon la revendication 4, **caractérisé en ce que** l'acrylate d'alkyle est l'acrylate d'éthyle.

6. Composition selon l'une quelconque des revendications 1 à 5, où le copolymère d'acide méthacrylique et d'acrylate d'alkyle en C₁-C₄ est réticulé par au moins un agent réticulant éthylèniquement polyinsaturé.

7. Composition selon la revendication 6, **caractérisée en ce que** la teneur en agent réticulant varie de 0,01 à 5% en poids et de préférence de 0,03 à 3% en poids et encore plus particulièrement de 0,05 à 1% en poids par rapport au poids total du copolymère.

8. Composition selon l'une quelconque des revendications 1 à 7, **caractérisée en ce que** le copolymère d'acide méthacrylique et d'acrylate d'alkyle en C₁-C₄ est sous forme de dispersion de particules dans l'eau.

9. Composition selon la revendication 8, **caractérisée en ce que** la taille moyenne en nombre des particules de copolymère dans la dispersion varie de 10 à 500 nm et de préférence de 20 à 200 nm et plus préférentiellement de 50 à 150 nm.

10. Composition selon l'une quelconque des revendications 1 à 9, **caractérisée par le fait que** les polyorganosiloxanes (i) sont choisis parmi ceux comportant :
a) des groupements aminés substitués ou non substitués ;
b) des groupements (per)fluorés ;
c) des groupements thiols ;
d) des groupements carboxylates ;
e) des groupements hydroxylés ;
f) des groupements alcoxylés ;
g) des groupements acyloxyalkyls ;
h) des groupements amphotères ;
i) des groupements bisulfites ;
j) des groupements hydroxyacylamino ;
k) des groupements acide carboxylique ou leur sels,
l) des groupements aryles.

11. Composition selon la revendication 10, **caractérisée en ce que** le polyorganosiloxane comportant des groupements aminés substitués ou non substitués est choisi parmi les amodiméthicones et les triméthylsilylamodiméthicones.

12. Composition selon l'une quelconque des revendications 1 à 11, **caractérisée en ce que** lesdits copolymères blocs linéaires polysiloxane(A)-polyoxyalkylène(B) de type (A-B)n répondent à la formule générale:
([Y (R₂SiO)ₐ R'₂SiYO][( CₙH₂ₙO)_{b}])_{c}
dans laquelle :
- R et R' identiques ou différents, représentent un radical hydrocarboné monovalent ne contenant pas d'insaturation aliphatique,
- n est un nombre entier compris entre 2 et 4,
- a est un nombre entier supérieur ou égal à 5,
- b est un nombre entier supérieur ou égal à 4,
- c est un nombre entier supérieur ou égal à 4,
- Y représente un groupe organique divalent qui est lié à l'atome de silicium adjacent par une liaison carbone-silicium et à un bloc polyoxyalkylène par un atome d'oxygène,
- le poids molaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids molaire moyen du copolymère bloc étant d'au moins 3000.

13. Composition selon l'une quelconque des revendications 1 à 12, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

14. Composition selon l'une quelconque des revendications précédentes, **caractérisée par le fait que** ledit polymère cationique est choisi parmi :
(1) les homopolymères ou copolymères dérivés d'esters ou d'amides acryliques ou méthacryliques et comportant au moins un des motifs de formules suivantes: 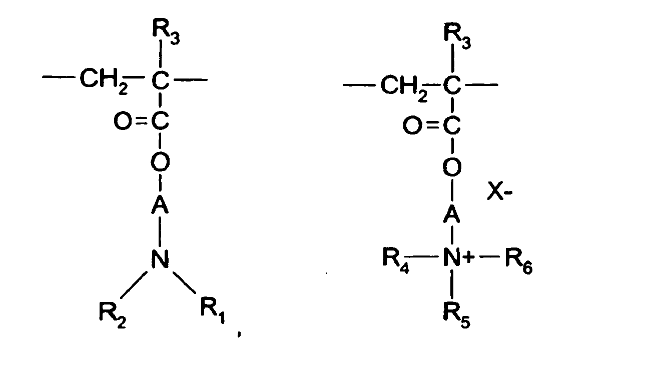 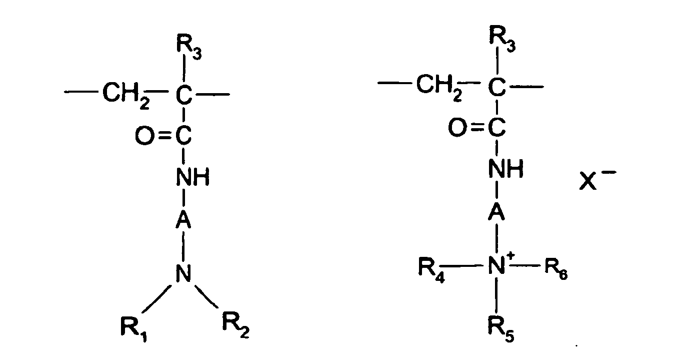 dans lesquelles:
R₃ , identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂ , identiques ou différents, représentent hydrogène ou un groupe alkyle ayant de 1 à 6 atomes de carbone;
X désigne un anion dérivé d'un acide minéral ou organique.
(2) Les polysaccharides cationiques,
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisées.
(5) les dérivés de polyaminoamides résultant de la condensation de polyalcoylènes polyamines avec des acides polycarboxyliques suivie d'une alcoylation par des agents bifonctionnels,
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(7) les cyclopolymères d'alkyl diallyl amine ou de dialkyl diallyl ammonium
(8) les polymères de diammonium quaternaire contenant des motifs récurrents répondant à la formule : 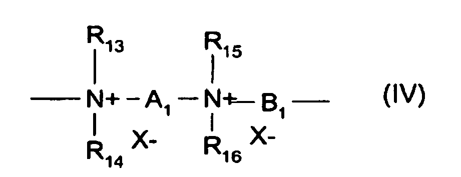 formule (IV) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;
De préférence, X⁻ est un anion.
(9) les polymères de polyammonium quaternaires comprenant des motifs de formule (VI): 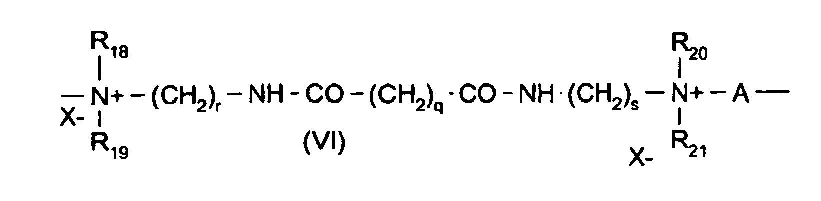 formule dans laquelle :
R18, R19, R20 et R21, identiques ou différents, représentent un atome d'hydrogène ou un radical méthyle, éthyle, propyle, β-hydroxyéthyle, β-hydroxypropyle ou
-CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(10) Les polymères quaternaires de vinylpyrrolidone et de vinylimidazole,
(11) Les polyamines référencées sous le nom de « POLYETHYLENEGLYCOL (15) TALLOW POLYAMINE » dans le dictionnaire CTFA.
(12) les polymères réticulés de sels de méthacryloyloxyalkyl(C₁-C₄) trialkyl(C₁₋C₄)ammonium,
(13) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

15. Composition selon la revendication précédente, **caractérisée par le fait que** ledit polymère cationique est choisi parmi les cyclopolymères cationiques, tes polysaccharides cationiques, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

16. Composition selon la revendication 15, **caractérisée par le fait que** ledit cyclopolymère est choisi parmi les homopolymères du chlorure de diallyldiméthylammonium et les copolymères du chlorure de diallyldiméthylammonium et d'acrylamide.

17. Composition selon la revendication 15, **caractérisée par le fait que** lesdits polysaccharides cationiques sont choisis parmi les gommes de guar modifiées par un sel de 2,3-époxypropyl triméthylammonium et les hydroxyéthylcelluloses ayant réagi avec un époxyde substitué par un groupement triméthylammonium.

18. Composition selon l'une quelconque des revendications 1 à 17, **caractérisée par le fait que** ledit polymère amphotère est choisi parmi :
(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. le composé vinylique peut être également un sel de dialkyldiallylammonium tel que le chlorure de diméthyldiallylammonium.
(2) les polymères comportant des motifs dérivant :
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quatemisation du méthacrylate de diméthylaminoéthyle avec le sulfate de diméthyle ou diéthyle.
(3) Les polyaminoamides réticulés et alcoylés partiellement ou totalement dérivant de polyaminoamides de formule générale :
-⁅CO-R₄-CO-Z⁆- (VII) 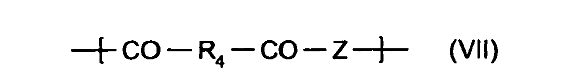
dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
a) dans les proportions de 60 à 100 moles %, le radical de formule (VIII) 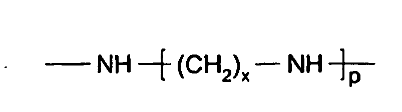 où x=2 et p=2 ou 3, ou bien x=3 et p=2
ce radical dérivant de la diéthylène triamine, de la triéthylène tétraamine ou de la dipropylène triamine;
b) dans les proportions de 0 à 40 moles % le radical (IV) ci-dessus, dans lequel x=2 et p=1 et qui dérive de l'éthylènediamine, ou le radical dérivant de la pipérazine : 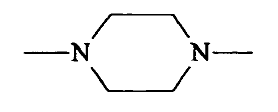
c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
(4) Les polymères comportant des motifs zwittérioniques de formule (IX): 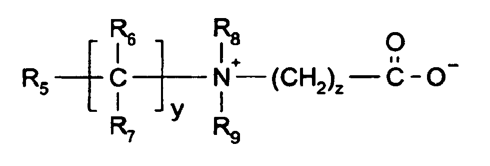 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
(5) les polymères dérivés du chitosane comportant des motifs monomères répondant aux formules (X), (XI), (XII) suivantes : 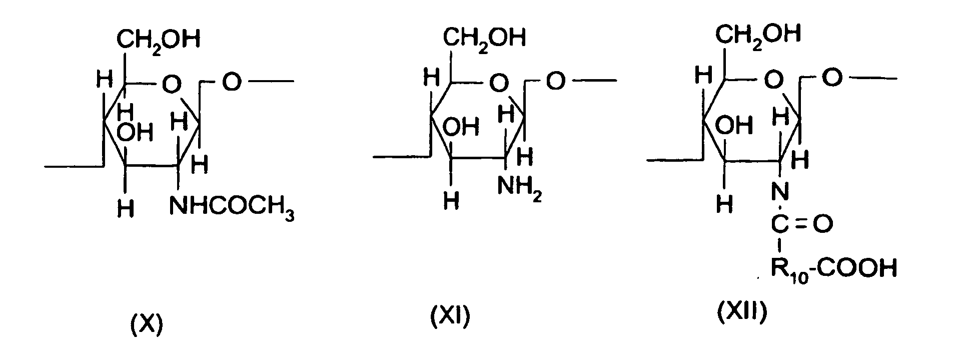 le motif (X) étant présent dans des proportions comprises entre 0 et 30%, le motif (XI) dans des proportions comprises entre 5 et 50% et le motif (XII) dans des proportions comprises entre 30 et 90%, étant entendu que dans ce motif (XII), R₁₀ représente un radical de formule: 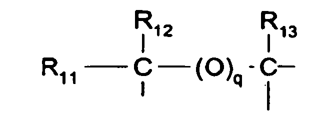 dans laquelle
si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XIII) tels que ceux décrits par exemple dans le brevet français 1 400 366 : 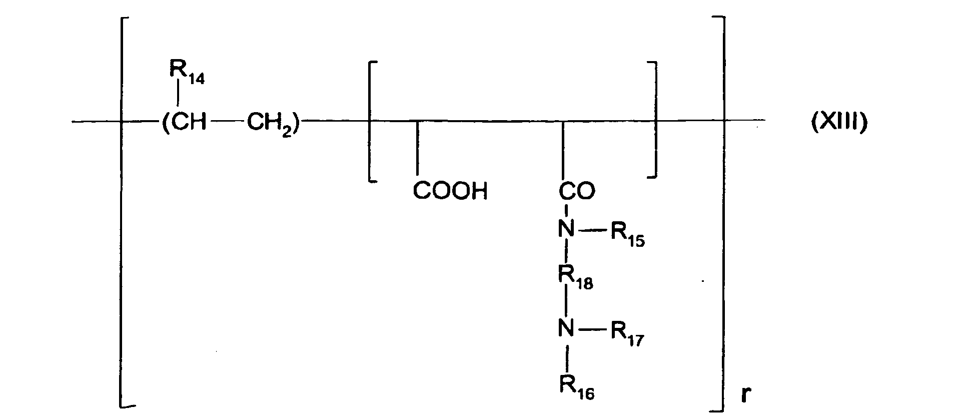 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂--CH₂-CH(CH₃)-, R₁₆ ayant les significations mentionnées ci-dessus, ainsi que les homologues supérieurs de ces radicaux et contenant jusqu'à 6 atomes de carbone.
(8) Des polymères amphotères du type -D-X-D-X- choisis parmi:
a) les polymères obtenus par action de l'acide chloracétique ou le chloracétate de sodium sur les composés comportant au moins un motif de formule :
-D-X-D-X-D- (XIV)
où D désigne un radical 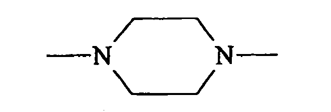 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
b) les polymères de formule :
-D-X-D-X- (XV)
où D désigne un radical 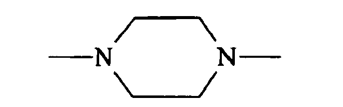 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine.

19. Composition selon l'une quelconque des revendications précédentes, **caractérisée en ce que** le copolymère acide méthacrylique et d'acrylate d'alkyle en C₁-C₄ est présent à une concentration comprise entre 0,01 % et 20 % en poids par rapport au poids total de la composition, de préférence entre 0,1 % et 5 % en poids.

20. Composition selon l'une quelconque des revendications précédentes, **caractérisée en ce que** la silicone est présente à une concentration comprise entre 0,001 % et 20 % en poids par rapport au poids total de la composition, de préférence entre 0,1 % et 10 % en poids.

21. Composition selon l'une quelconque des revendications précédentes, **caractérisée en ce que** le polymère cationique ou amphotère est présent à une concentration comprise entre 0,001 % et 20 % en poids par rapport au poids total de la composition, de préférence entre 0,01 % et 10 % en poids.

22. Composition selon l'une quelconque des revendications 1 à 21, **caractérisée par le fait qu'**elle comprend en outre au moins une silicone différente des silicones telles que définies dans les revendications 1 et 10 à 12.

23. Composition selon la revendication 22, **caractérisée par le fait que** les silicones sont des polyorganosiloxanes non volatils choisis parmi les polyalkylsiloxanes, les gommes et résines de silicones, ainsi que leurs mélanges.

24. Composition selon la revendication 23, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les gommes de silicone sont choisies parmi les polydiorganosiloxanes ayant des masses molaires moyennes en nombre comprises entre 200 000 et 1 000 000 utilisés seuls ou sous forme de mélange dans un solvant ;
(d) les résines sont choisies parmi les résines comprenant des unités : R₃ Si O_{1/2}, R₂ Si Oi_{2/2}, R Si O_{3/2}, Si O_{4/2}
dans lesquelles R représente un groupement hydrocarboné ayant de 1 à 16 atomes de carbone.

25. Composition selon rune quelconque des revendications 22 à 24, **caractérisée par le fait que** les silicones additionnelles sont choisies parmi les polyalkylsiloxanes à groupements terminaux triméthylsilyle, les polyalkylsiloxanes à groupements terminaux diméthylsilanol, les mélanges de deux PDMS constitués d'une gomme et d'une huile de viscosités différentes, les mélanges d'organosiloxanes et de silicones cycliques, les résines d'organopolysiloxanes.

26. Composition selon l'une quelconque des revendications 1 à 25, **caractérisée par le fait qu'**elle comprend en outre au moins un agent bénéfique pour les matières kératiniques choisi parmi les esters d'acides carboxyliques en C1-C30 et de d'alcools mono ou polyhydroxylés en C1-C30, les huiles végétales, animales, minérales ou de synthèse, les cires, les céramides, les pseudocéramides.

27. Composition selon l'une quelconque des revendications 22 à 26, **caractérisée en ce que** ladite silicone additionnelle différente des silicones définies aux revendications 1 et 10 à 12 représente de 0,001 % à 20 % en poids par rapport au poids total de la composition, de préférence entre 0,01 % à 10 % en poids.

28. Composition selon la revendication 26, **caractérisée en ce que** l'agent bénéfique pour les matières kératiniques représente de 0.001 % à 20 % en poids par rapport au poids total de la composition, de préférence entre 0,01 % à 10 % en poids.

29. Composition selon l'une quelconque des revendications précédentes, **caractérisée par le fait qu**'elle comprend en outre au moins un agent tensioactif choisi parmi les tensioactifs anioniques, non ioniques, amphotères, cationiques et leurs mélanges.

30. Composition selon la revendication 29, **caractérisée par le fait que** le ou les agents tensioactifs sont choisis parmi au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

31. Composition selon l'une quelconque des revendications 29 ou 30, **caractérisée par le fait que** le ou les agents tensioactifs sont présents à une concentration comprise entre 0,01% et 50% en poids, de préférence entre 0,1% et 40% en poids, et encore plus préférentiellement entre 0,5% et 30% en poids, par rapport au poids total de la composition.

32. Composition selon l'une quelconque des revendications 1 à 31, **caractérisée par le fait** la composition contient au moins un additif choisi parmi les épaississants, les agents antipelliculaires ou anti-séborrhéiques, les parfums, les agents nacrants, les hydroxyacides, les électrolytes, les esters d'acides gras, les conservateurs, les filtres solaires siliconés ou non, les vitamines, les provitamines telles que le panthénol, les polymères anioniques ou non ioniques non siliconés, les protéines non cationiques_{,} les hydrolysats de protéines non cationiques, l'acide méthyl-18 eicosanoique, les huiles fluorées ou perfluorées, les amines grasses, les acides gras et leurs dérivés, les alcools gras et leurs dérivés ainsi que les mélanges de ces différents composés. non cationiques

33. Composition selon l'une quelconque des revendications précédentes, **caractérisée par le fait qu**'elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

34. Utilisation d'une composition telle que définie dans l'une quelconque des revendications précédentes pour le lavage ou pour le soin des matières kératiniques.

35. Procédé de traitement des matières kératiniques, telles que les cheveux, **caractérisé en ce qu**'il consiste à appliquer sur lesdites matières une composition cosmétique selon l'une quelconque des revendications 1 à 33, puis à effectuer éventuellement un rinçage à l'eau.

36. Utilisation d'une composition telle que définie dans l'une quelconque des revendications 1 à 33 pour apporter de la brillance aux cheveux.

37. Utilisation d'une composition telle que définie dans l'une quelconque des revendications 1 à 33 pour apporter de la légèreté aux cheveux.

38. Utilisation d'une composition telle que définie dans l'une quelconque des revendications 1 à 33 pour apporter de la douceur aux cheveux.

39. Utilisation d'une composition telle que définie dans l'une quelconque des revendications 1 à 33 pour apporter, du lissage au toucher aux cheveux.

40. Utilisation d'une composition telle que définie dans l'une quelconque des revendications 1 à 33 pour apporter de la souplesse aux cheveux.

## Claims

1. Cosmetic composition, **characterized in that** it comprises, in a cosmetically acceptable medium, at least one crosslinked copolymer of methacrylic acid and of a C₁-C₄ alkyl acrylate, at least one cationic or amphoteric polymer and at least one silicone chosen from:
(i) polyorganosiloxanes comprising in their general structure one or more organofunctional groups directly attached to the siloxane chain or attached via a hydrocarbon radical, with the exclusion of soluble silicones chosen from polydimethylsiloxanes comprising polyoxyethylenated end groups and/or side groups;
(ii) linear polysiloxane (A)-polyoxyalkylene (B) block copolymers of (A-B)n type with n >3;
(iii) grafted silicone polymers, with a non-silicone organic skeleton, consisting of a main organic chain formed from organic monomers not comprising silicone; onto which is grafted, into the said chain and also optionally on at least one of its ends, at least one polysiloxane macromonomer;
(iv) grafted silicone polymers, with a polysiloxane skeleton grafted with non-silicone organic monomers, comprising a main polysiloxane chain onto which is grafted, into the said silicone chain and also optionally on at least one of its ends, at least one organic macromonomer not comprising silicone;
(v) or mixtures thereof.

2. Composition according to Claim 1, **characterized in that**, in the said copolymer, the methacrylic acid is present in amounts ranging from 20% to 80% by weight and more particularly from 25% to 70% by weight and more particularly still from 35% to 60% by weight relative to the total weight of the copolymer.

3. Composition according to Claim 1 or 2, **characterized in that**, in the said copolymer, the alkyl acrylate is present in amounts ranging from 15% to 80% by weight and more particularly from 25% to 75% by weight and more particularly still from 40% to 65% by weight relative to the total weight of the copolymer.

4. Composition according to any one of Claims 1 to 3, **characterized in that**, in the said copolymer, the alkyl acrylate is chosen from methyl acrylate, ethyl acrylate or butyl acrylate.

5. Composition according to Claim 4, **characterized in that** the alkyl acrylate is ethyl acrylate.

6. Composition according to any one of Claims 1 to 5, wherein the copolymer of methacrylic acid and of C₁-C₄ alkyl acrylate is crosslinked with at least one polyethylenically unsaturated crosslinking agent.

7. Composition according to Claim 6, **characterized in that** the amount of crosslinking agent varies from 0.01% to 5% by weight and preferably from 0.03% to 3% by weight and still more particularly from 0.05% to 1% by weight relative to the total weight of the copolymer.

8. Composition according to any one of Claims 1 to 7, **characterized in that** the copolymer of methacrylic acid and of C₁-C₄ alkyl acrylate is in the form of a dispersion of particles in water.

9. Composition according to Claim 8, **characterized in that** the number-average size of the copolymer particles in the dispersion varies from 10 to 500 nm and preferably from 20 to 200 nm and more preferably from 50 to 150 nm.

10. Composition according to any one of Claims 1 to 9, **characterized in that** the polyorganosiloxanes (i) are chosen from those comprising:
a) substituted or unsubstituted amine groups;
b) (per)fluoro groups;
c) thiol groups;
d) carboxylate groups;
e) hydroxylated groups;
f) alkoxy groups;
g) acyloxyalkyl groups;
h) amphoteric groups;
i) bisulphite groups;
j) hydroxyacylamino groups;
k) carboxylic acid groups or salts thereof
l) aryl groups.

11. Composition according to Claim 10, **characterized in that** the polyorganosiloxane comprising substituted or unsubstituted amine groups is chosen from amodimethicones and trimethylsilylamodimethicones.

12. Composition according to any one of Claims 1 to 11, **characterized in that** the said linear polysiloxane(A)-polyoxyalkylene(B) block copolymers of (A-B)n type correspond to the general formula:
([Y(R₂SiO)ₐR'₂SiYO] [(CₙH₂ₙO)_{b}])_{c} (I)
in which
- R and R', which may be identical or different, represent a monovalent hydrocarbon radical not containing aliphatic unsaturation,
- n is an integer ranging from 2 to 4,
- a is an integer greater than or equal to 5,
- b is an integer greater than or equal to 4,
- c is an integer greater than or equal to 4,
- Y is a divalent organic group that is linked to the adjacent silicon atom via a carbon-silicon bond and to a polyoxyalkylene block via an oxygen atom,
- the average molecular weight of each siloxane block is between about 400 and about 10 000, that of each polyoxyalkylene block being between about 300 and about 10 000,
- the siloxane blocks represent from about 10% to about 95% of the weight of the block copolymer,
- the average molecular weight of the block copolymer is at least 3 000.

13. Composition according to any one of Claims 1 to 12, **characterized in that** the cationic polymers are chosen from those containing units comprising primary, secondary, tertiary and/or quaternary amine groups that may either form part of the main polymer chain, or may be borne by a side substituent directly attached to the said chain.

14. Composition according to any one of the preceding claims, **characterized in that** the said cationic polymer is chosen from:
(1) homopolymers or copolymers derived from acrylic or methacrylic esters or amides and comprising at least one of the units of the following formulae: 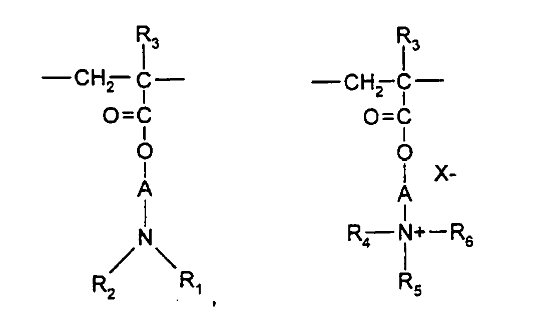 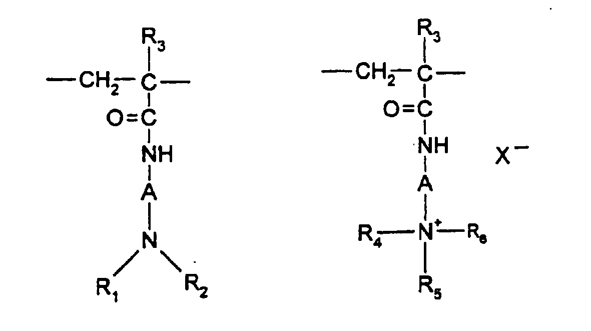 in which:
R₃, which may be identical or different, denote a hydrogen atom or a CH₃ radical;
A, which may be identical or different, represent a linear or branched alkyl group of 1 to 6 carbon atoms, or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from an inorganic or organic acid.
(2) cationic polysaccharides,
(3) polymers consisting of piperazinyl units and of divalent alkylene or hydroxyalkylene radicals containing straight or branched chains, optionally interrupted with oxygen, sulphur or nitrogen atoms or with aromatic or heterocyclic rings, and also the products of oxidation and/or quaternization of these polymers,
(4) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent is used in proportions ranging from 0.025 to 0.35 mol per amine group of the polyamino amide; these polyamino amides can be alkylated or, if they contain one or more tertiary amine functions, quaternized.
(5) polyamino amide derivatives resulting from the condensation of polyalkylene polyamines with polycarboxylic acids followed by an alkylation with difunctional agents,
(6) polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms,
(7) cyclopolymers of alkyldiallylamine or of dialkyldiallylammonium,
(8) quaternary diammonium polymers containing repeating units corresponding to the formula: 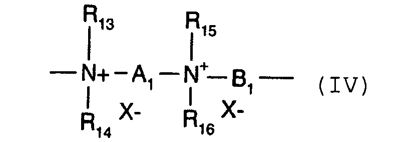 in which formula (IV):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second hetero atom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms, which groups may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
- (CH₂-CH₂-O)ₓ-CH₂-CH₂-
- [CH₂-CH (CH₃) -O]_{y}-CH₂-CH (CH₃) -
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula:
- NH-Y-NH-, where Y denotes a linear or branched hydrocarbon radical, or alternatively the divalent radical
- CH₂-CH₂-S-S-CH₂-CH₂-;
d) a ureylene group of formula: -NH-CO-NH-;
preferably, X⁻ is an anion,
(9) polyquaternary ammonium polymers consisting of units of formula (VI): 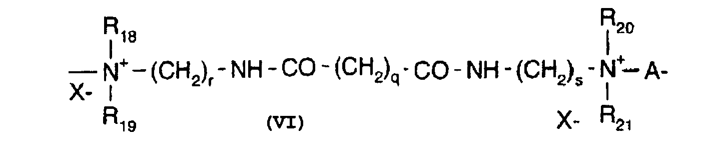 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-,
(10) quaternary polymers of vinylpyrrolidone and of vinylimidazole,
(11) the polyamines referenced under the name "Polyethylene glycol (15) tallow polyamine" in the CTFA dictionary;
(12) crosslinked polymers of methacryloyloxy-(C₁-C₄)alkyltri(C₁-C₄)alkylammonium salts,
(13) polyalkyleneimines, in particular polyethyleneimines, polymers containing vinylpyridine or vinylpyridinium units, condensates of polyamines and of epichlorohydrin, quaternary polyureylenes and chitin derivatives.

15. Composition according to the preceding claim, **characterized in that** the said cationic polymer is chosen from cationic cyclopolymers, cationic polysaccharides, quaternary polymers of vinylpyrrolidone and of vinylimidazole, crosslinked homopolymers or copolymers of methacryloyloxy (C₁-C₄) alkyltri (C₁-C₄) alkylammonium salts, and mixtures thereof.

16. Composition according to Claim 15, **characterized in that** the said cyclopolymer is chosen from diallyldimethylammonium chloride homopolymers and copolymers of diallyldimethylammonium chloride and of acrylamide.

17. Composition according to Claim 15, **characterized in that** the said cationic polysaccharides are chosen from guar gums modified with a 2,3-epoxypropyltrimethylammonium salt and hydroxyethylcelluloses that have reacted with an epoxide substituted with a trimethylammonium group.

18. Composition according to any one of Claims 1 to 17, **characterized in that** the said amphoteric polymer is chosen from:
(1) polymers resulting from the copolymerization of a monomer derived from a vinyl compound bearing a carboxylic group such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylate and acrylate, dialkylaminoalkylmethacrylamide and -acrylamide; the vinyl compound may also be a dialkyldiallylammonium salt such as dimethyldiallylammonium chloride,
(2) polymers containing units derived from:
a) at least one monomer chosen from acrylamides and methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulphate,
(3) crosslinked and alkylated polyamino amides partially or totally derived from polyamino amides of general formula:
-⁅CO-R₄-CO-Z⁆- (VII)
in which R₄ represents a divalent radical derived from a saturated dicarboxylic acid, a mono- or dicarboxylic aliphatic acid containing an ethylenic double bond, an ester of a lower alkanol, having 1 to 6 carbon atoms, of these acids or a radical derived from the addition of any one of the said acids to a bis (primary) or bis(secondary) amine, and Z denotes a bis(primary), mono- or bis(secondary) polyalkylene-polyamine radical and preferably represents:
a) in proportions of from 60 to 100 mol%, the radical of formula (VIII) 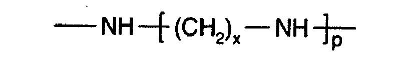 where x = 2 and p = 2 or 3, or alternatively x = 3 and p = 2
this radical being derived from diethylenetriamine, from triethylenetetraamine or from dipropylenetriamine;
b) in proportions of from 0 to 40 mol%, the radical (IV) above in which x = 2 and p = 1 and which is derived from ethylenediamine, or the radical derived from piperazine: 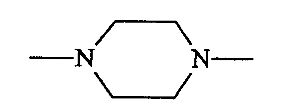
c) in proportions of from 0 to 20 mol%, the -NH-(CH₂)₆-NH- radical derived from hexamethylenediamine, these polyamino amines being crosslinked by addition of a difunctional crosslinking agent chosen from epihalohydrins, diepoxides, dianhydrides and bis-unsaturated derivatives, using from 0.025 to 0.35 mol of crosslinking agent per amine group of the polyamino amide and alkylated by the action of acrylic acid, chloroacetic acid or an alkane sultone, or salts thereof,
(4) polymers containing zwitterionic units of formula (IX): 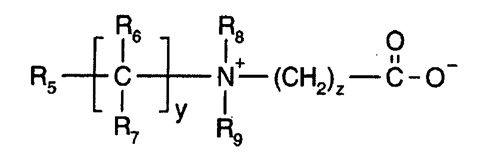 in which R₅ denotes a polymerizable unsaturated group such as an acrylate, methacrylate, acrylamide or methacrylamide group, y and z represent an integer from 1 to 3, R₆ and R₇ represent a hydrogen atom, methyl, ethyl or propyl, R₈ and R₉ represent a hydrogen atom or an alkyl radical such that the sum of the carbon atoms in R₈ and R₉ does not exceed 10,
(5) polymers derived from chitosan containing monomer units corresponding to formulae (X), (XI) and (XII) below: 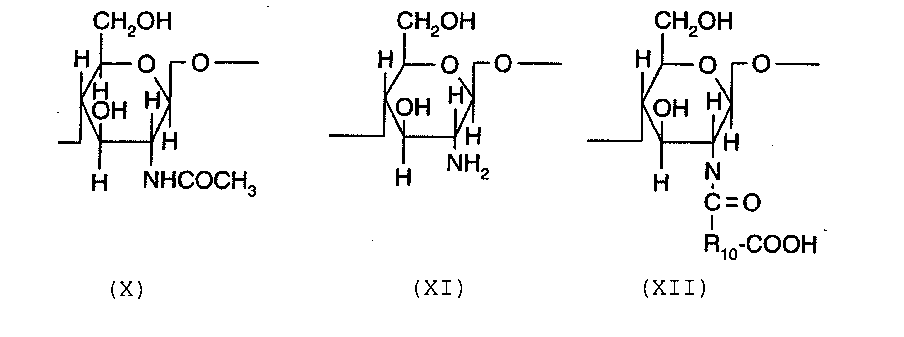 the unit (X) being present in proportions of between 0 and 30%, the unit (XI) in proportions of between 5% and 50% and the unit (XII) in proportions of between 30% and 90%, it being understood that, in this unit (XII), R₁₀ represents a radical of formula: 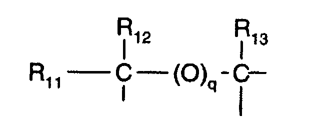 in which
if q = 0, R₁₁, R₁₂ and R₁₃, which may be identical or different, each represent a hydrogen atom, a methyl, hydroxyl, acetoxy or amino residue, a monoalkylamine residue or a dialkylamine residue which are optionally interrupted by one or more nitrogen atoms and/or optionally substituted with one or more amine, hydroxyl, carboxyl, alkylthio or sulphonic groups, an alkylthio residue in which the alkyl group bears an amino residue, at least one of the radicals R₁₁, R₁₂ and R₁₃ being, in this case, a hydrogen atom;
or, if q = 1, R₁₁, R₁₂ and R₁₃ each represent a hydrogen atom, and also the salts formed by these compounds with bases or acids.
(6) polymers derived from the N-carboxyalkylation of chitosan, such as N-carboxymethylchitosan or N-carboxybutylchitosan.
(7) polymers corresponding to the general formula (XIII) as described, for example, in French patent 1 400 366: 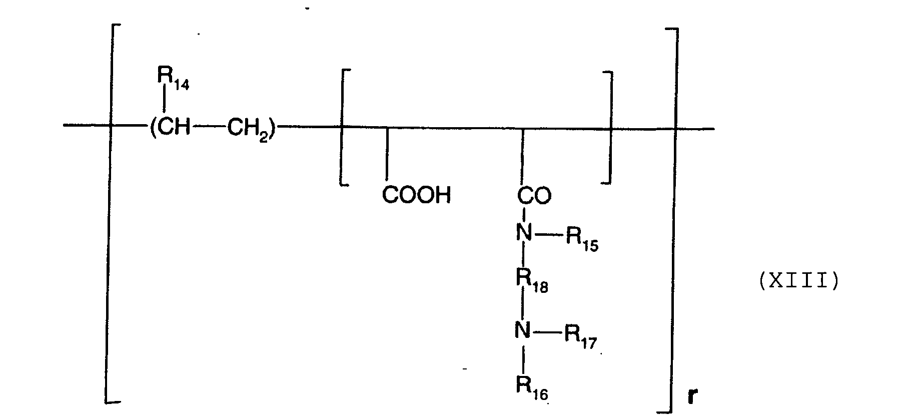 in which R₁₄ represents a hydrogen atom, a CH₃O, CH₃CH₂O or phenyl radical, R₁₅ denotes hydrogen or a lower alkyl radical such as methyl or ethyl, R₁₆ denotes hydrogen or a lower alkyl radical such as methyl or ethyl, R₁₇ denotes a lower alkyl radical such as methyl or ethyl or a radical corresponding to the formula: -R₁₈-N (R₁₆)₂, R₁₈ representing a -CH₂-CH₂-, -CH₂-CH₂-CH₂- or -CH₂-CH (CH₃)- group, R₁₆ having the meanings mentioned above,
and also the higher homologues of these radicals and containing up to 6 carbon atoms,
(8) amphoteric polymers of the type -D-X-D-X-chosen from:
a) polymers obtained by the action of chloroacetic acid or sodium chloroacetate on compounds containing at least one unit of formula:
-D-X-D-X-D- (XIV)
where D denotes a radical 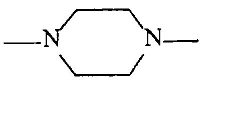 and X denotes the symbol E or E', E or E', which may be identical or different, denote a divalent radical which is an alkylene radical with a straight or branched chain containing up to 7 carbon atoms in the main chain, which is unsubstituted or substituted with hydroxyl groups and which can contain, in addition to the oxygen, nitrogen and sulphur atoms, 1 to 3 aromatic and/or heterocyclic rings; the oxygen, nitrogen and sulphur atoms being present in the form of ether, thioether, sulphoxide, sulphone, sulphonium, alkylamine or alkenylamine groups, hydroxyl, benzylamine, amine oxide, quaternary ammonium, amide, imide, alcohol, ester and/or urethane groups;
b) polymers of formula:
-D-X-D-X- (XV)
where D denotes a radical 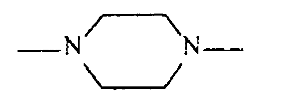 and X denotes the symbol E or E' and at least once E'; E having the meaning given above and E' being a divalent radical which is an alkylene radical with a straight or branched chain having up to 7 carbon atoms in the main chain, which is unsubstituted or substituted with one or more hydroxyl radicals and containing one or more nitrogen atoms, the nitrogen atom being substituted with an alkyl chain which is optionally interrupted by an oxygen atom and necessarily containing one or more carboxyl functions or one or more hydroxyl functions and betainized by reaction with chloroacetic acid or sodium chloroacetate.
(9) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkanolamine.

19. Composition according to any one of the preceding claims, **characterized in that** the copolymer of methacrylic acid and of a C₁-C₄ alkyl acrylate is present in a concentration of between 0.01% and 20% by weight relative to the total weight of the composition and preferably between 0.1% and 5% by weight.

20. Composition according to any one of the preceding claims, **characterized in that** the silicone is present in a concentration of between 0.001% and 20% by weight relative to the total weight of the composition and preferably between 0.1% and 10% by weight.

21. Composition according to any one of the preceding claims, **characterized in that** the cationic or amphoteric polymer is present in a concentration of between 0.001% and 20% by weight relative to the total weight of the composition and preferably between 0.01% and 10% by weight.

22. Composition according to any one of Claims 1 to 21, **characterized in that** it also comprises at least one silicone other than silicones as defined in Claims 1 and 10 to 12.

23. Composition according to Claim 22, **characterized in that** the silicones are non-volatile polyorganosiloxanes chosen from polyalkylsiloxanes and silicone gums and resins, and also mixtures thereof.

24. Composition according to Claim 23, **characterized in that**:
(a) the polyalkylsiloxanes are chosen from:
- polydimethylsiloxanes containing trimethylsilyl end groups;
- polydimethylsiloxanes containing dimethylsilanol end groups;
- poly (C₁-C₂₀) alkylsiloxanes;
(b) the silicone gums are chosen from polydiorganosiloxanes with number-average molar masses of between 200 000 and 1 000 000, used alone or in the form of a mixture in a solvent;
(d) the resins are chosen from resins consisting of units: R₃ Si O_{1/2}, R₂ Si O_{2/2}, R Si O_{3/2}, Si O_{4/2}
in which R represents a hydrocarbon group containing from 1 to 16 carbon atoms.

25. Composition according to any one of Claims 22 to 24, **characterized in that** the additional silicones are chosen from polyalkylsiloxanes containing trimethylsilyl end groups, polyalkylsiloxanes containing dimethylsilanol end groups, mixtures of two PDMSs consisting of a gum and an oil of different viscosities, mixtures of organosiloxanes and of cyclic silicones, and organopolysiloxane resins.

26. Composition according to any one of Claims 1 to 25, **characterized in that** it also comprises at least one agent that is beneficial for keratih materials, chosen from esters of C₁-C₃₀ carboxylic acids and of C₁-C₃₀ mono- or polyhydroxylated alcohols, plant, animal, mineral or synthetic oils, waxes, ceramides and pseudoceramides.

27. Composition according to any one of Claims 22 to 26, **characterized in that** the said additional silicone other than the silicones defined in Claims 1 and 10 to 12 represents from 0.001% to 20% by weight relative to the total weight of the composition and preferably between 0.1% to 10% by weight.

28. Composition according to Claim 26, **characterized in that** the agent that is beneficial for keratin materials represents from 0.001% to 20% by weight relative to the total weight of the composition and preferably between 0.01% and 10% by weight.

29. Composition according to any one of the preceding claims, **characterized in that** it also comprises at least one surfactant chosen from anionic, nonionic, amphoteric and cationic surfactants, and mixtures thereof.

30. Composition according to Claim 29, **characterized in that** the surfactant(s) is (are) chosen from at least one or more anionic surfactants or mixtures of at least one or more anionic surfactants and of at least one or more amphoteric surfactants or of at least one or more nonionic surfactants.

31. Composition according to either of Claims 29 and 30, **characterized in that** the surfactant(s) is (are) present in a concentration of between 0.01% and 50% by weight, preferably between 0.1% and 40% by weight and even more preferably between 0.5% and 30% by weight relative to the total weight of the composition.

32. Composition according to any one of Claims 1 to 31, **characterized in that** the composition contains at least one additive chosen from thickeners, antidandruff agents, anti-seborrhoeic agents, fragrances, nacreous agents, hydroxy acids, electrolytes, fatty acid esters, preserving agents, silicone or non-silicone sunscreens, vitamins, provitamins such as panthenol, non-silicone anionic or nonionic polymers, non-cationic proteins, non-cationic protein hydrolysates, 18-methyleicosanoic acid, fluoro or perfluoro oils, fatty amines, fatty acids and derivatives thereof, fatty alcohols and derivatives thereof, and also mixtures of these various compounds.

33. Composition according to any one of the preceding claims, **characterized in that** it is in the form of a shampoo, a conditioner, a permanent-waving, straightening, dyeing or bleaching composition for the hair, a rinse-out composition to be applied between the two steps of a permanent-waving or hair-straightening operation, or a washing composition for the body.

34. Use of a composition as defined in any one of the preceding claims, for washing or caring for keratin materials.

35. Process for treating keratin materials, such as the hair, **characterized in that** it consists in applying to the said materials a cosmetic composition according to any one of Claims 1 to 33, and then optionally rinsing it out with water.

36. Use of a composition as defined in any one of Claims 1 to 33 for imparting sheen to the hair.

37. Use of a composition as defined in any one of Claims 1 to 33 for imparting lightness to the hair.

38. Use of a composition as defined in any one of Claims 1 to 33 for imparting softness to the hair.

39. Use of a composition as defined in any one of Claims 1 to 33 for imparting smooth feel to the hair.

40. Use of a composition as defined in any one of Claims 1 to 33 for imparting suppleness to the hair.

## Patentansprüche

1. Kosmetische Zusammensetzung, **dadurch gekennzeichnet, dass** sie in einem kosmetisch akzeptablen Medium mindestens ein vernetztes Copolymer von Methacrylsäure und C₁₋₄-Alkylacrylat, mindestens ein kationisches oder amphoteres Polymer und mindestens ein Silicon enthält, das ausgewählt ist unter:
(i) Polyorganosiloxanen, die in ihrer allgemeinen Struktur eine oder mehrere organofunktionelle Gruppen aufweisen, die direkt oder über eine Kohlenwasserstoffgruppe an die Siloxankette gebunden sind, wobei lösliche Silicone ausgenommen sind, die unter den Polydimethylsiloxanen ausgewählt sind, die endständige und/oder als Seitengruppen vorliegende polyethoxylierte Gruppen aufweisen;
(ii) linearen Blockcopolymeren Polysiloxan(A)-polyoxyalkylen(B) vom Typ (A-B)ₙ mit n > 3;
(iii) gepfropften Siliconpolymeren mit nicht siliconiertem organischen Grundgerüst, die aus einer organischen Hauptkette bestehen, die aus organischen Monomeren gebildet wird, die kein Silicon enthalten, und auf die im Inneren der Kette sowie gegebenenfalls an mindestens einem Kettenende mindestens ein Polysiloxanmakromer gepfropft ist;
(iv) gepfropften Siliconpolymeren mit Polysiloxangrundgerüst, auf das nicht siliconierte organische Monomere gepfropft sind, die eine Polysiloxanhauptkette aufweisen, auf die im Inneren der Kette sowie gegebenenfalls an mindestens einem Kettenende mindestens ein organisches Makromonomer, das kein Silicon enthält, gepfropft ist;
(v) oder deren Gemischen.

2. Zusammensetzung nach Anspruch 1, **dadurch gekennzeichnet, dass** die Methacrylsäure in dem Copolymer in Mengenanteilen von 20 bis 80 Gew.-%, insbesondere 25 bis 70 Gew.-% und besonders 35 bis 60 Gew.-%, bezogen auf das Gesamtgewicht des Copolymers, vorliegt.

3. Zusammensetzung nach Anspruch 1 oder 2, **dadurch gekennzeichnet, dass** das Alkylacrylat in dem Copolymer in Mengenanteilen von 15 bis 80 Gew.-%, insbesondere 25 bis 75 Gew.-% und besonders 40 bis 65 Gew.-%, bezogen auf das Gesamtgewicht des Copolymers, enthalten ist.

4. Zusammensetzung nach einem der Ansprüche 1 bis 3, **dadurch gekennzeichnet, dass** in dem Copolymer das Alkylacrylat unter Methylacrylat, Ethylacrylat oder Butylacrylat ausgewählt ist.

5. Zusammensetzung nach Anspruch 4, **dadurch gekennzeichnet, dass** das Alkylacrylat das Ethylacrylat ist.

6. Zusammensetzung nach einem der Ansprüche 1 bis 5, wobei das Copolymer von Methacrylsäure und C₁₋₄-Alkylacrylat mit mindestens einem mehrfach ethylenisch ungesättigten Vernetzungsmittel vernetzt ist.

7. Zusammensetzung nach Anspruch 6, **dadurch gekennzeichnet, dass** der Mengenanteil des Vernetzungsmittels im Bereich von 0,01 bis 5 Gew.-%, vorzugsweise 0,03 bis 3 Gew.-% und insbesondere 0,05 bis 1 Gew.-%, bezogen auf das Gesamtgewicht des Copolymers, liegt.

8. Zusammensetzung nach einem der Ansprüche 1 bis 7, **dadurch gekennzeichnet, dass** das Copolymer von Methacrylsäure und C₁₋₄-Alkylacrylat in Form einer Dispersion von Partikeln in Wasser vorliegt.

9. Zusammensetzung nach Anspruch 8, **dadurch gekennzeichnet, dass** die zahlenmittlere Größe der Copolymerpartikel in Dispersion im Bereich von 10 bis 500 nm, vorzugsweise 20 bis 200 nm und besonders bevorzugt 50 bis 150 nm liegt.

10. Zusammensetzung nach einem der Ansprüche 1 bis 9, **dadurch gekennzeichnet, dass** die Polyorganosiloxane (i) unter den Verbindungen ausgewählt sind, die enthalten:
a) substituierte oder nicht substituierte aminierte Gruppen;
b) (per)fluorierte Gruppen;
c) Thiolgruppen;
d) Carboxylatgruppen;
e) hydroxylierte Gruppen;
f) alkoxylierte Gruppen;
g) Acyloxyalkylgruppen;
h) amphotere Gruppen;
i) Bisulfitgruppen;
j) Hydroxyacylaminogruppen;
k) Carbonsäuregruppen oder deren Salze;
l) Arylgruppen.

11. Zusammensetzung nach Anspruch 10, **dadurch gekennzeichnet, dass** das Polyorganosiloxan, das substituierte oder unsubstituierte aminierte Gruppen enthält, unter den Amodimethiconen und Trimethylsilylamodimethiconen ausgewählt ist.

12. Zusammensetzung nach einem der Ansprüche 1 bis 11, **dadurch gekennzeichnet, dass** die linearen Blockcopolymere Polysiloxan(A)-polyoxyalkylen(B) vom Typ (A-B)ₙ der folgenden allgemeinen Formel entsprechen:
([Y (R₂SiO)ₐ R'₂SiYO][(CₙH₂ₙO)]_{b})_{c}
worin bedeuten:
- die Gruppen R und R', die gleich oder verschieden sind, eine einwertige Kohlenwasserstoffgruppe, die keine aliphatische ungesättigte Bindung enthält,
- n eine ganze Zahl von 2 bis 4,
- a eine ganze Zahl von 5 oder darüber,
- b eine ganze Zahl von 4 oder darüber,
- c eine ganze Zahl von 4 oder darüber,
- Y eine zweiwertige organische Gruppe, die an das angrenzende Siliciumatom über eine Kohlenstoff-Siliciumbindung und an einen Polyoxyalkylenblock über ein Sauerstoffatom gebunden ist,
- wobei die mittlere Molmasse jedes Siloxanblocks im Bereich von etwa 400 bis etwa 10 000 und die jedes Polyoxyalkylenblocks im Bereich von etwa 300 bis etwa 10 000 liegt,
- wobei die Siloxanblöcke etwa 10 bis etwa 95 Gew.-% des Blockcopolymers ausmachen und
- wobei die mittlere Molmasse des Blockcopolymers mindestens 3 000 beträgt.

13. Zusammensetzung nach einem der Ansprüche 1 bis 12, **dadurch gekennzeichnet, dass** die kationischen Polymere unter den Polymeren ausgewählt sind, die Einheiten enthalten, die primäre, sekundäre, tertiäre und/oder quartäre Aminogruppen aufweisen, die entweder Teil der Polymerhauptkette sein können oder von einem direkt an die Polymerhauptkette gebundenen seitlichen Substituenten getragen werden.

14. Zusammensetzung nach einem der vorhergehenden Ansprüche, **dadurch gekennzeichnet, dass** das kationische Polymere ausgewählt ist:
(1) Homopolymeren oder Copolymeren, die von Acrylsäureestern, Methacrylsäureestern, Acrylamiden oder Methacrylamiden ausgewählt sind, die mindestens eine Einheit der folgenden Formeln enthalten: 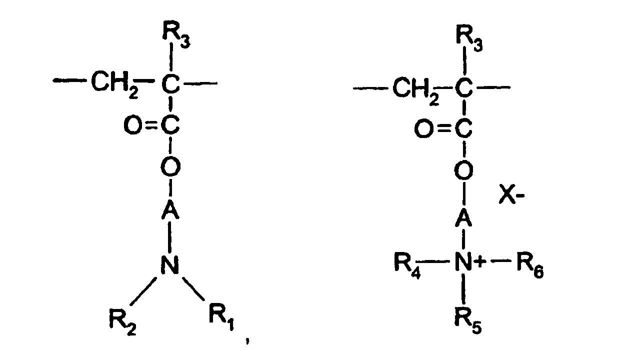 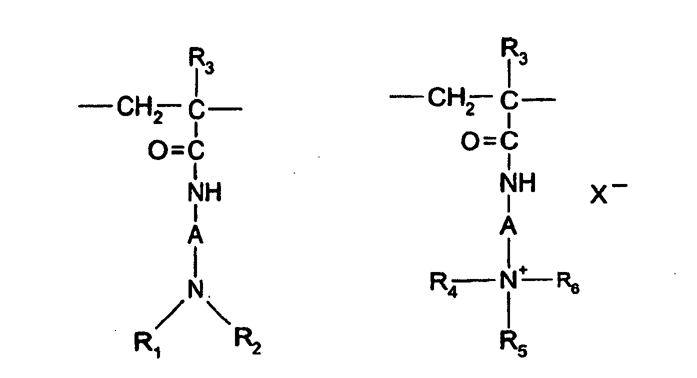 worin bedeuten:
die Gruppen R₃, die gleich oder verschieden sind, ein Wasserstoffatom oder die Gruppe CH₃;
die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen;
die Gruppe R₄, R₅ oder R₆, die gleich oder verschieden sind, eine Alkylgruppe mit 1 bis 18 Kohlenstoffatomen und eine Benzylgruppe;
die Gruppen R₁ und R₂, die gleich oder verschieden sind, ein Wasserstoffatom oder eine Alkylgruppe mit 1 bis 6 Kohlenstoffatomen;
X ein von einer anorganischen oder organischen Säure abgeleitetes Anion;
(2) kationischen Polysacchariden;
(3) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder durch aromatische oder heterocyclische Ringe unterbrochen sind, sowie den Oxidationsprodukten und/oder Quaternisierungsprodukten dieser Polymere;
(4) in Wasser löslichen Polyaminoamiden, die insbesondere durch Polykondensation einer sauren Verbindung mit einem Polyamin hergestellt werden; wobei diese Polyaminoamide mit einem Epihalohydrin, Diepoxid, Dianhydrid, ungesättigtem Dianhydrid, zweifach ungesättigten Derivat, Bis-halohydrin, Bis-azetidinium, Bis-haloacyldiamin, Alkyl-bis-halogenid oder auch einem Oligomer vernetzt sein können, das bei der Reaktion einer bifunktionellen Verbindung gebildet wird, das gegenüber einem Bis-halohydrin, Bis-azetidinium, Bis-haloacyldiamin, Alkyl-bis-halogenid, Epihalohydrin, Diepoxid oder einem zweifach ungesättigten Derivat reaktiv ist; wobei das Vernetzungsmittel in Mengenanteilen von 0,025 bis 0,35 mol pro Aminogruppe des Polyaminoamids verwendet wird; wobei die Polyaminoamide alkyliert, oder, wenn sie eine oder mehrere tertiäre Aminogruppen enthalten, quaternisiert sein können;
(5) Polyaminoamidderivaten, die bei der Kondensation von Po-lyalkylenpolyaminen mit Polycarbonsäuren und anschließender Alkylierung mit bifunktionellen Stoffen gebildet werden;
(6) Polymeren, die durch Umsetzung eines Polyalkylenpolyamins mit zwei primären Aminogruppen und mindestens einer sekundären Aminogruppe mit einer Dicarbonsäure erhalten werden, die unter Diglycolsäure und den gesättigten aliphatischen Dicarbonsäuren mit 3 bis 8 Kohlenstoffatomen ausgewählt ist;
(7) Alkyldiallylamincyclopolymeren oder Dialkyldiallylammoniumcyclopolymeren;
(8) quartären Diammnoiumpolymeren, die wiederkehrende Einheiten der folgenden Formel enthalten: 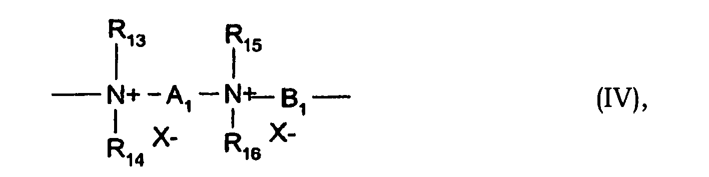 wobei in der Formel (IV):
die Gruppen R₁₃, R₁₄, R₁₅ und R₁₆, die gleich oder verschieden sind, aliphatische, alicyclische oder arylaliphatische Gruppen mit 1 bis 20 Kohlenstoffatomen oder niedere hydroxyalkylaliphatische Gruppen bedeuten oder die Gruppen R₁₃, R₁₄, R₁₅ und R₁₆ gemeinsam oder getrennt voneinander mit den Stickstoffatomen, an die sie gebunden sind, Heterocyclen bilden, die gegebenenfalls ein von Stickstoff verschiedenes, zweites Heteroatom enthalten, oder die Gruppen R₁₃, R₁₄, R₁₅ und R₁₆ eine geradkettige oder verzweigte C₁-₆-Alkylgruppe bedeuten, die mit einer Gruppe Nitril, Ester, Acyl, Amid oder -CO-O-R₁₇-D oder -CO-NH-R₁₇-D substituiert ist, wobei R₁₇ eine Alkylengruppe ist und D eine quartäre Ammoniumgruppe bedeutet;
A₁ und B₁ Polymethylengruppen mit 2 bis 20 Kohlenstoffatomen bedeuten, die geradkettig oder verzweigt, gesättigt oder ungesättigt vorliegen können und an die Hauptkette gebunden oder in der Hauptkette einen oder mehrere aromatische Ringe oder ein oder mehrere Sauerstoffatome, Schwefelatome oder Gruppen Sulfoxid, Sulfon, Disulfid, Amino, Alkylamino, Hydroxy, quartäres Ammonium, Ureido, Amid oder Ester enthalten können, und
X⁻ ein von einer anorganischen oder organischen Säure abgeleitetes Anion;
A₁, R₁₃ und R₁₅ mit den beiden Stickstoffatomen, an die sie gebunden sind, einen Piperazinring bilden können; wenn A₁ eine geradkettige oder verzweigte, gesättigte oder ungesättigte Alkylen- oder Hydroxyalkylengruppe bedeutet, kann B₁ auch eine Gruppe -(CH₂)ₙ-CO-D-OC-(CH₂)ₙ- bedeuten, worin D bedeutet:
a) einen Glycolrest der Formel -O-Z-O-, wobei Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃-O]_{y}-CH₂-CH(CH₃)-
wobei x und y eine ganze Zahl von 1 bis 4 bedeuten und einen wohldefinierten und einzigen Polymerisationsgrad angeben oder eine beliebige Zahl von 1 bis 4 und einen mittleren Polymerisationsgrad bedeuten;
b) einen bis-sekundären Diaminrest, wie ein Piperazinderivat;
c) einen bis-primären Diaminrest der Formel -NH-Y-NH-, wobei Y eine geradkettige oder verzweigte Kohlenwasserstoffgruppe bedeutet, oder die zweiwertige Gruppe
-CH₂-CH₂-S-S-CH₂-CH₂-;
d) eine Ureylengruppe der folgenden Formel: -NH-CO-NH-; wobei X⁻ vorzugswesie ein Anion ist;
(9) quartären Polyammoniumpolymeren, die Einheiten der folgenden Formel (VI) enthalten: 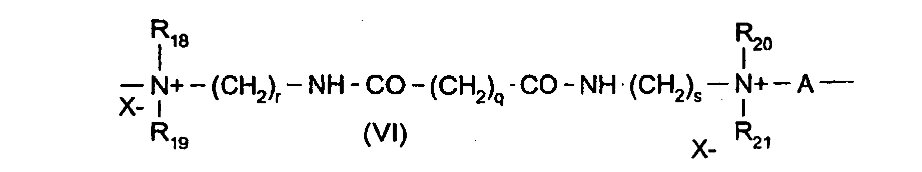 wobei in der Formel bedeuten:
R₁₈, R₁₉, R₂₀ und R₂₁, die gleich oder verschieden sind, ein Wasserstoffatom oder Methyl, Ethyl, Propyl, β-Hydroxyethyl, β-Hydroxypropyl oder
-CH₂CH₂(OCH₂CH₂)ₚOH,
wobei p 0 oder eine ganze Zahl von 1 bis 6 bedeutet, mit der Maßgabe, dass R₁₈, R₁₉, R₂₀ und R₂₁ nicht gleichzeitig Wasserstoff bedeuten,
r und s, die gleich oder verschieden sind, ganze Zahlen von 1 bis 6,
q 0 oder eine ganze Zahl von 1 bis 34,
X ein Halogenatom,
A eine Dihalogenidgruppe oder vorzugsweise
-CH₂-CH₂-O-CH₂-CH₂-;
(10) quartären Polymeren von Vinylpyrrolidon und Vinylimidazol;
(11) Polyaminen, die nach CTFA-Nomenklatur als "POLYETHYLENGLYCOL (15) TALLOW POLYAMINE" bezeichnet werden;
(12) vernetzten Polymeren von Methacryloyloxyalkyl(C₁₋₄)trialkyl(C₁₋₄)ammoniumsalzen;
(13) Polyalkyleniminen, insbesondere Polyethyleniminen, Polymeren, die Vinylpyridin- oder Vinylpyridiniumeinheiten enthalten, Kondensaten von Polyaminen und Epichlorhydrin, quartären Polyureylenen und Chitinderivaten.

15. Zusammensetzung nach dem vorhergehenden Anspruch, **dadurch gekennzeichnet, dass** das kationische Polymer unter den kationischen Cyclopolymeren, kationischen Polysacchariden, quartären Polymeren von Vinylpyrrolidon und Vinylimidazol, vernetzten Homopolymeren oder Copolymeren von Methacryloyloxyalkyl(C₁₋₄)trialkyl(C₁₋₄)ammoniumsalzen und deren Gemischen ausgewählt ist.

16. Zusammensetzung nach Anspruch 15, **dadurch gekennzeichnet, dass** das Cyclopolymer unter den Homopolymeren von Diallyldimethylammoniumchlorid und Copolymeren von Diallyldimethylammoniumchlorid und Acrylamid ausgewählt ist.

17. Zusammensetzung nach Anspruch 15, **dadurch gekennzeichnet, dass** die kationischen Polysaccharide unter den Guargummen, die mit einem 2,3-Epoxypropyltrimethylammoniumsalz modifiziert sind, und Hydroxyethylcellulosen ausgewählt sind, die mit einem mit einer Trimethylammoniumgruppe substituierten Epoxid umgesetzt wurden.

18. Zusammensetzung nach einem der Ansprüche 1 bis 17, **dadurch gekennzeichnet, dass** das amphotere Polymer ausgewählt ist unter:
(1) Polymeren, die bei der Copolymerisation eines Monomers, das von einer Vinylverbindung abgeleitet ist, die eine Carboxygruppe aufweist, wie insbesondere Acrylsäure, Methacrylsäure, Maleinsäure, α-Chloracrylsäure, und eines basischen Monomers gebildet wird, das von einer substituierten Vinylverbindung abgeleitet ist, die mindestens ein basisches Atom aufweist, wie insbesondere Dialkylaminoalkylmethacrylaten und -acrylaten, Dialkylaminoalkylmethacrylamiden und -acrylamiden, wobei die Vinylverbindung auch ein Dialkyldiallylammoniumsalz wie Dimethyldiallylammoniumchlorid bedeuten kann:
(2) Polymeren, die Einheiten aufweisen, die abgeleitet sind:
a) zumindest einem Monomer, das unter den Acrylamiden oder Methacrylamiden ausgewählt ist, die am Stickstoff mit einer Alkylgruppe substituiert sind,
b) mindestens einem sauren Comonomer, das eine oder mehrere reaktive Carboxygruppen enthält, und
c) mindestens einem basischen Comonomer, wie Estern von Acrylsäure und Methacrylsäure mit primären, sekundären, tertiären und quartären Amminosubstituenten und dem Quaternisierungsprodukt von Dimethylaminoethylmethacrylat mit Dimethylsulfat oder Diethylsulfat;
(3) ganz oder teilweise alkylierten und vernetzten Polyaminoamiden, die von Polyaminoamiden der folgenden allgemeinen Formel abgeleitet sind:
-⁅CO-R₄-CO-Z⁆- (VII)
worin R₄ eine zweiwertige Gruppe bedeutet, die von einer gesättigten Dicarbonsäure, einer aliphatischen Mono- oder Dicarbonsäure mit ethylenischer Doppelbindung, einem Ester eines niederen Alkanols mit 1 bis 6 Kohlenstoffatomen dieser Säuren oder einer Gruppe abgleitet ist, die bei der Addition einer dieser Säuren und einem bis-primären oder bis-sekundären Amin gebildet wird, und Z eine bis-primäre, mono- oder bis-sekundäre Polyalkylenpolyamingruppe ist, und vorzugsweise:
a) in Mengenanteilen von 60 bis 100 Mol-% die Gruppe der Formel (VIII) 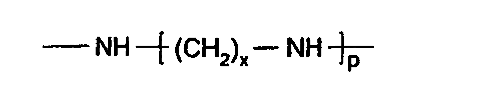 mit x = 2 und p = 2 oder 3 oder x = 3 und p = 2 wobei diese Gruppe von Diethylentriamin, Triethylentetramin oder Dipropylentriamin stammt;
b) in Anteilen von 0 bis 40 Mol-% die Gruppe (IV) mit x = 2 und p = 1, die von Ethylendiamin abgeleitet ist, oder die von Piperazin abgeleitete Gruppe: 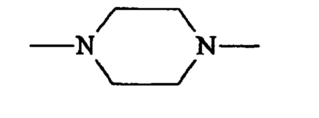
c) in Anteilen von 0 bis 20 Mol-% die Gruppe -NH-(CH₂)₆-NH-, die von Hexamethylendiamin stammt,
wobei die Polyaminoamine durch Zugabe eines bifunktionellen Vernetzungsmittels, das unter den Epihalohydrinen, Diepoxiden, Dianhydriden, zweifach ungesättigten Derivaten ausgewählt ist, in einer Menge von 0,025 bis 0,35 mol Vernetzungsmittel pro Aminogruppe des Polyaminoamids vernetzt sind und durch Zugabe von Acrylsäure, Chloressigsäure oder einem Alkansulton oder deren Salzen alkyliert ist;
(4) Polymeren, die zwitterionische Einheiten der Formel (IX) enthalten: 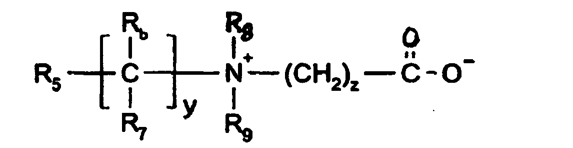 worin R₅ eine polymerisierbare ungesättigte Gruppe bedeutet, wie Acrylat, Methacrylat, Acrylamid oder Methacrylamid, y und z eine ganze Zahl von 1 bis 3 bedeuten, R₆ und R₇ ein Wasserstoffatom, Methyl, Ethyl oder Propyl bedeuten, R₈ und R₉ ein Wasserstoffatom oder eine Alkylgruppe bedeuten, die so gewählt ist, dass die Summe der Kohlenstoffatome in R₈ und R₉ 10 nicht übersteigt;
(5) Polymeren, die von Chitosan abgeleitet sind und Monomereinheiten der folgenden Formeln (X), (XI) und (XII) enthalten: 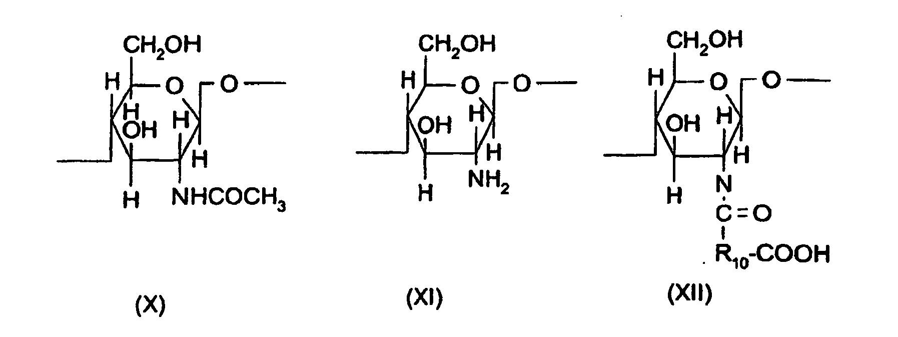 wobei die Einheit (X) in Anteilen von 0 bis 30 %, die Einheit (XI) in Anteilen von 5 bis 50 % und die Einheit (XII) in anteilen von 30 bis 90 % enthalten ist, mit der Maßgabe, dass in der Einheit (XII) R₁₀ eine Gruppe der folgenden Formel bedeutet: 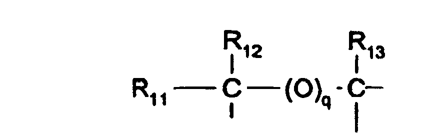 worin bedeuten:
wenn q = 0, die Gruppen R₁₁, R₁₂ und R₁₃, die identisch oder voneinander verschieden sind, jeweils ein Wasserstoffatom, Methyl, Hydroxy, Acetoxy oder Amino, einen Monoalkylaminrest oder Dialkylaminrest, die gegebenenfalls durch ein oder mehrere Stickstoffatome unterbrochen und/oder gegebenenfalls mit einer oder mehreren Gruppen Amin, Hydroxy, Carboxy, Alkylthio, Sulfonsäure substituiert sind, einen Alkylthiorest, worin die Alkylgruppe eine Aminogruppe trägt, wobei mindestens eine der Gruppen R₁₁, R₁₂ und R₁₃ in diesem Fall ein Wasserstoffatom bedeutet;
oder wenn q = 1, die Gruppen R₁₁, R₁₂ und R₁₃ jeweils ein Wasserstoffatom, sowie den Salzen, die diese Verbindungen mit Basen oder Säuren bilden;
(6) Polymeren, die bei der N-Carboxyalkylierung von Chitosan entstehen, wie N-Carboxymethylchitosan oder N-Carboxybutylchitosan;
(7) Polymeren, die der allgemeinen Formel (XIII) entsprechen und die beispielsweise in dem französischen Patent 1 400 366 beschrieben wurden: 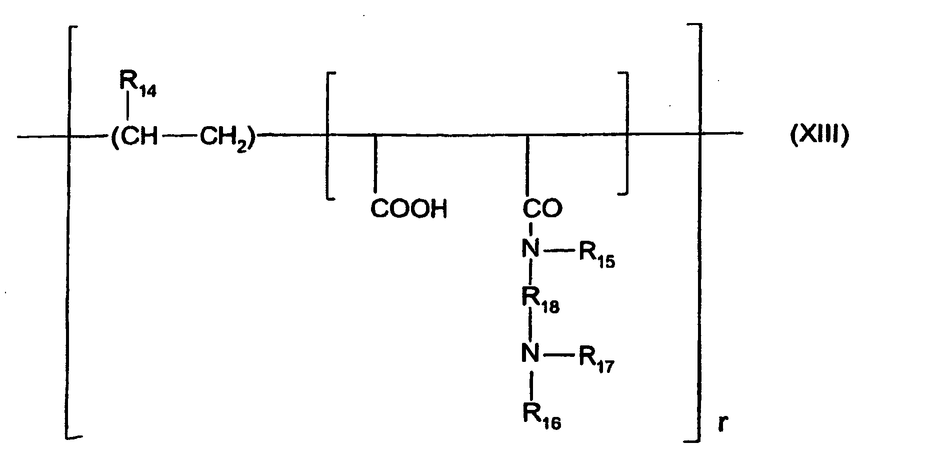 worin R₁₄ ein Wasserstoffatom, CH₃O, CH₃CH₂O, Phenyl bedeutet, R₁₅ ein Wasserstoffatom oder eine niedere Alkylgruppe wie Methyl, Ethyl ist, R₁₆ ein Wasserstoffatom oder eine niedere Alkylgruppe wie Methyl, Ethyl bedeutet, R₁₇ eine niedere Alkylgruppe bedeutet, wie Methyl, Ethyl, oder eine Gruppe der Formel: -R₁₈-N(R₁₆)₂, wobei R₁₈ eine Gruppe -CH₂-CH₂-, -CH₂-CH₂-CH₂-, -CH₂-CH(CH₃)- ist und R₁₆ die oben angegebenen Bedeutungen aufweist,
sowie die höheren Homologen dieser Gruppen, die bis zu 6 Kohlenstoffatome enthalten;
(8) amphoteren Polymeren vom Typ -D-X-D-X-, die ausgewählt sind unter:
a) Polymeren, die bei der Umsetzung von Chloressigsäure oder Natriumchloracetat mit Verbindungen erhalten werden, die mindestens eine Einheit der folgenden Formel enthalten:
-D-X-D-X-D- (XIV)
wobei D eine Gruppe 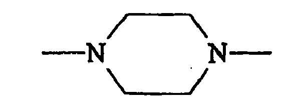 und X die Symbole E oder E' bedeutet, wobei E oder E', die gleich oder verschieden sind, eine zweiwertige Gruppe bedeuten, bei der es sich um eine Alkylengruppe mit gerader oder verzweigter Kette mit bis zu 7 Kohlenstoffatomen in der Hauptkette handelt, die unsubstituiert vorliegt oder mit Hydroxygruppen substituiert ist und ferner Sauerstoffatome, Stickstoffatome, Schwefelatome, 1 bis 3 aromatische und/oder heteroaromatische Ringe enthalten kann; wobei die Sauerstoffatome, Stickstoffatome und Schwefelatome in der Form der folgenden Gruppen enthalten sein können: Ether, Thioether, Sulfoxid, Sulfon, Sulfonium, Alkylamin, Alkenylamin, Hydroxy, Benzylamin, Aminoxid, quartäre Ammoniumgruppen, Amid, Imid, Alkohol, Ester und/oder Urethan;
b) Polymeren der Formel:
-D-X-D-X- (XV)
wobei D eine Gruppe 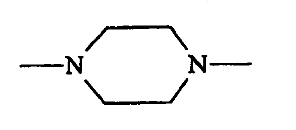 und X die Symbole E oder E' und mindestens einmal E' und X die Symbole E oder E' und mindestens einmal E' bedeutet; wobei E die oben angegebenen Bedeutungen aufweist und E' eine zweiwertige Gruppe ist, bei der es sich um eine Alkylengruppe mit gerader oder verzweigter Kette und bis zu 7 Kohlenstoffatomen in der Hauptkette handelt, die unsubstituiert vorliegt oder mit einer oder mehreren Hydroxygruppen substituiert ist und ein oder mehrere Stickstoffatome enthält, wobei das Stickstoffatom mit einer Alkylenkette substituiert ist, die gegebenenfalls durch ein Sauerstoffatom unterbrochen ist und zwingend eine oder mehrere Carboxyfunktionen oder eine oder mehrere Hydroxyfunktionen enthält und durch Umsetzung mit Chloressigsäure oder Natriumchloracetat betainisiert wurde;
(9) Copolymeren Alkyl(C₁₋₅)vinylether/Maleinsäureanhydrid, das partiell durch Semiamidierung mit einem N,N-Dialkylaminoalkylamin wie N,N-Dimethylaminopropylamin oder Semiveresterung mit einem N,N-Dialkanolamin modifiziert wurde.

19. Zusammensetzung nach einem der vorhergehenden Ansprüche, **dadurch gekennzeichnet, dass** das Copolymer von Methacrylsäure und C₁₋₄-Acrylacrylat in einer Konzentration von 0,01 bis 20 Gew.-%, bezogen auf das Gesamtgewicht der Zusammensetzung, und vorzugsweise 0,1 bis 5 Gew.-% enthalten ist.

20. Zusammensetzung nach einem der vorhergehenden Ansprüche, **dadurch gekennzeichnet, dass** das Silicon in einer Konzentration von 0,001 bis 20 Gew.-%, bezogen auf das Gesamtgewicht der Zusammensetzung, und vorzugsweise 0,1 bis 10 Gew.-% enthalten ist.

21. Zusammensetzung nach einem der vorhergehenden Ansprüche, **dadurch gekennzeichnet, dass** das kationische oder amphotere Polymer in einer Konzentration von 0,001 bis 20 Gew.-%, bezogen auf das Gesamtgewicht der Zusammensetzung, und vorzugsweise 0,01 bis 10 Gew.-% enthalten ist.

22. Zusammensetzung nach einem der Ansprüche 1 bis 21, **dadurch gekennzeichnet, dass** sie ferner mindestens ein Silicon enthält, das von den in den Ansprüchen 1 und 10 bis 12 definierten Siliconen verschieden ist.

23. Zusammensetzung nach Anspruch 22, **dadurch gekennzeichnet, dass** die Silicone nichtflüchtige Polyorganosiloxane sind, die unter den Polyalkylsiloxanen, Silicongummis und Siliconharzen sowie deren Gemischen ausgewählt sind.

24. Zusammensetzung nach Anspruch 23, **dadurch gekennzeichnet, dass**
(a) die Polyalkylsiloxane ausgewählt sind unter:
- Polydimethylsiloxanen mit endständigen Trimethylsilylgruppen;
- Polydimethylsiloxanen mit endständigen Dimethylsilanolgruppen;
- Polyalkyl(C₁₋₂₀)siloxanen;
(b) die Silicongummis ausgewählt sind unter Polydiorganosiloxanen, die zahlenmittlere Molmassen von 200 000 bis 1 000 000 aufweisen und alleine oder in Form eines Gemisches in einem Lösungsmittel verwendet werden;
(d) die Harze ausgewählt sind unter den Harzen, die Einheiten: R₃SiO_{1/2}, R₂SiO_{2/2}, RSiO_{3/2}, SiO_{4/2} enthalten, worin R eine Kohlenwasserstoffgruppe mit 1 bis 16 Kohlenstoffatomen bedeutet.

25. Zusammensetzung nach einem der Ansprüche 22 bis 24, **dadurch gekennzeichnet, dass** die zusätzlichen Silicone unter den Polyalkylsiloxanen mit endständigen Trimethylsilylgruppen, Polyalkylsiloxanen mit endständigen Dimethylsilanolgruppen, Gemischen aus zwei PDMS, die aus einem Gummi und einem Öl mit unterschiedlichen Viskositäten bestehen, Gemischen von Organosiloxanen und cyclischen Siliconen, Organopolysiloxanharzen ausgewählt sind.

26. Zusammensetzung nach einem der Ansprüche 1 bis 25, **dadurch gekennzeichnet, dass** sie ferner mindestens einen Stoff enthält, der eine günstige Wirkung auf die Keratinsubstanzen aufweist und unter den Estern von Carbonsäuren mit 1 bis 30 Kohlenstoffatomen und ein- oder mehrwertigen Alkoholen mit 1 bis 30 Kohlenstoffatomen, pflanzlichen Ölen, tierischen Ölen, Mineralölen oder synthetischen Ölen, Wachsen, Ceramiden oder Pseudoceramiden ausgewählt ist.

27. Zusammensetzung nach einem der Ansprüche 22 bis 26, **dadurch gekennzeichnet, dass** das von den in den Ansprüchen 1 und 10 bis 12 definierten Siliconen verschiedene, zusätzliche Silicon 0,001 bis 20 Gew.-%, bezogen auf das Gesamtgewicht der Zusammensetzung, und vorzugsweise 0,01 bis 10 Gew.-% ausmacht.

28. Zusammensetzung nach Anspruch 26, **dadurch gekennzeichnet, dass** der für Keratinsubstanzen günstige Stoff 0,001 bis 20 Gew.-%, bezogen auf das Gesamtgewicht der Zusammensetzung, und vorzugsweise 0,01 bis 10 Gew.-% ausmacht.

29. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren, kationischen grenzflächenaktiven Stoffen und deren Gemischen ausgewählt ist.

30. Zusammensetzung nach Anspruch 29, **dadurch gekennzeichnet, dass** der oder die grenzflächenaktive(n) Stoff(e) unter mindestens einem oder mehreren anionischen grenzflächenaktiven Stoffen oder Gemischen von mindestens einem oder mehreren anionischen grenzflächenaktiven Stoffen und mindestens einem oder mehreren amphoteren grenzflächenaktiven Stoffen oder mindestens einem oder mehreren nichtionischen grenzflächenaktiven Stoffen ausgewählt sind.

31. Zusammensetzung nach einem der Ansprüche 29 oder 30, **dadurch gekennzeichnet, dass** der oder die grenzflächenaktiven Stoffe in einer Konzentration von 0,01 bis 50 Gew.-%, vorzugsweise 0,1 bis 40 Gew.-% und noch bevorzugter 0,5 bis 30 Gew.-%, bezogen auf das Gesamtgewicht der Zusammensetzung, enthalten sind.

32. Zusammensetzung nach einem der Ansprüche 1 bis 31, **dadurch gekennzeichnet, dass** die Zusammensetzung mindestens einen Zusatzstoff enthält, der unter den Verdickungsmitteln, Antischuppenmitteln, Wirkstoffen gegen Seborrhoe, Parfums, Perlglanzstoffen, Hydroxysäuren, Elektrolyten, Fettsäureestern, Konservierungsmitteln, siliconierten oder nicht siliconierten Sonnenschutzfiltern, Vitaminen, Provitaminen wie Panthenol, anionischen oder nichtionischen, nicht siliconierten Polymeren, nicht kationischen Proteinen, nicht kationischen Proteinhydrolysaten, 18-Methyleicosansäure, fluorierten oder perfluorierten Ölen, Fettaminen, Fettsäuren und deren Derivaten, Fettalkoholen und deren Derivaten sowie Gemischen dieser verschiedenen Verbindungen ausgewählt ist.

33. Zusammensetzung nach einem der vorhergehenden Ansprüche, **dadurch gekennzeichnet, dass** sie als Haarwaschmittel, Produkt, das nach der Haarwäsche angewandet wird, Zusammensetzung für dauerhafte Verformungen, Entkräuselungen, Färbungen oder Entfärbungen der Haare, Zusammensetzung, die ausgespült wird und zwischen den beiden Schritten einer dauerhaften Verformung oder Entkräuselung aufgetragen wird, oder reinigende Zusammensetzung für den Körper vorliegt.

34. Verwendung einer Zusammensetzung nach einem der vorhergehenden Ansprüche für die Reinigung oder die Pflege von Keratinsubstanzen.

35. Verfahren zur Behandlung von Keratinsubstanzen, wie Haaren, **dadurch gekennzeichnet, dass** es darin besteht, auf die Keratinsubstanzen eine kosmetische Zusammensetzung nach einem der Ansprüche 1 bis 33 aufzutragen und anschließend gegebenenfalls mit Wasser zu spülen.

36. Verwendung einer Zusammensetzung nach einem der Ansprüche 1 bis 33, um den Haaren Glanz zu geben.

37. Verwendung einer Zusammensetzung nach einem der Ansprüche 1 bis 33, damit die Haare locker werden.

38. Verwendung einer Zusammensetzung nach einem der Ansprüche 1 bis 33, um den Haaren Weichheit zu geben.

39. Verwendung einer Zusammensetzung nach einem der Ansprüche 1 bis 33, damit sich die Haare glatt anfühlen.

40. Verwendung einer Zusammensetzung nach einem der Ansprüche 1 bis 33, um den Haaren Geschmeidigkeit zu geben.
